# EUROPEAN PATENT APPLICATION

(11) **EP 3 254 691 A1**
(43) Date of publication of application: **13.12.2017**
(21) Application number: 16305664.1
(22) Date of filing: 07.06.2016
(51) Int. Cl.: A61K 39/12, C07K 16/06, C07K 16/10

(54) **POLYCLONAL ANTIBODIES FOR USE IN THE PREVENTION AND/OR TREATMENT OF EBOLA VIRUS DISEASE**

(71) Applicant: ABIVAX, 75008 Paris (FR)
(72) Inventor: CRABE, Sandrine, 34980 Saint-Gély-du-Fesc (FR); DENIS, Jérôme, 34980 Saint-Clément-de-Rivière (FR); FANGET, Bernard, 42800 Châteauneuf (FR)
(74) Representative: Cabinet Becker et Associés

(57) **Abstract**

The present invention relates to polyclonal antibodies for use in the prevention and/or treatment of the Ebola virus disease. In one embodiment, the polyclonal antibodies are specific for a truncated Ebola virus GP glycoprotein. Said polyclonal antibodies are preferably non-human antibodies and are provided in a serum free pharmaceutical composition. The present invention also relates to a method of prevention and/or treatment of Ebola virus disease in a subject in need thereof, comprising administering to said subject polyclonal antibodies specific for the Ebola virus.

SEQUENCE LISTING

<110>  ABIVAX

<120>  POLYCLONAL ANTIBODIES FOR USE IN THE PREVENTION AND/OR TREATMENT
       OF EBOLA VIRUS DISEASE

<130>  BET15P2625

<160>  11

<170>  PatentIn version 3.5

<210>  1
<211>  676
<212>  PRT
<213>  Artificial Sequence

<220>
<223>  GP consensus sequence

<400>  1

Met Gly Val Thr Gly Ile Leu Gln Leu Pro Arg Asp Arg Phe Lys Arg
1               5                   10                  15

Thr Ser Phe Phe Leu Trp Val Ile Ile Leu Phe Gln Arg Thr Phe Ser
            20                  25                  30

Ile Pro Leu Gly Val Ile His Asn Ser Thr Leu Gln Val Ser Asp Val
        35                  40                  45

Asp Lys Leu Val Cys Arg Asp Lys Leu Ser Ser Thr Asn Gln Leu Arg
    50                  55                  60

Ser Val Gly Leu Asn Leu Glu Gly Asn Gly Val Ala Thr Asp Val Pro
65                  70                  75                  80

Ser Val Thr Lys Arg Trp Gly Phe Arg Ser Gly Val Pro Pro Lys Val
                85                  90                  95

Val Asn Tyr Glu Ala Gly Glu Trp Ala Glu Asn Cys Tyr Asn Leu Glu
            100                 105                 110

Ile Lys Lys Pro Asp Gly Ser Glu Cys Leu Pro Ala Ala Pro Asp Gly
        115                 120                 125

Ile Arg Gly Phe Pro Arg Cys Arg Tyr Val His Lys Val Ser Gly Thr
    130                 135                 140

Gly Pro Cys Ala Gly Asp Phe Ala Phe His Lys Glu Gly Ala Phe Phe
145                 150                 155                 160

Leu Tyr Asp Arg Leu Ala Ser Thr Val Ile Tyr Arg Gly Thr Thr Phe
                165                 170                 175

Ala Glu Gly Val Val Ala Phe Leu Ile Leu Pro Gln Ala Lys Lys Asp
            180                 185                 190

Phe Phe Ser Ser His Pro Leu Arg Glu Pro Val Asn Ala Thr Glu Asp
        195                 200                 205

Pro Ser Ser Gly Tyr Tyr Ser Thr Thr Ile Arg Tyr Gln Ala Thr Gly
    210                 215                 220

Phe Gly Thr Asn Glu Thr Glu Tyr Leu Phe Glu Val Asp Asn Leu Thr
225                 230                 235                 240

Tyr Val Gln Leu Glu Ser Arg Phe Thr Pro Gln Phe Leu Leu Gln Leu
                245                 250                 255

Asn Glu Thr Ile Tyr Ala Ser Gly Lys Arg Ser Asn Thr Thr Gly Lys
            260                 265                 270

Leu Ile Trp Lys Val Asn Pro Glu Ile Asp Thr Thr Ile Gly Glu Trp
        275                 280                 285

Ala Phe Trp Glu Thr Lys Lys Asn Leu Thr Arg Lys Ile Arg Ser Glu
    290                 295                 300

Glu Leu Ser Phe Thr Ala Val Ser Asn Gly Pro Lys Asn Ile Ser Gly
305                 310                 315                 320

Gln Ser Pro Ala Arg Thr Ser Ser Asp Pro Glu Thr Asn Thr Thr Asn
                325                 330                 335

Glu Asp His Lys Ile Met Ala Ser Glu Asn Ser Ser Ala Met Val Gln
            340                 345                 350

Val His Ser Gln Gly Arg Lys Ala Ala Val Ser His Leu Thr Thr Leu
        355                 360                 365

Ala Thr Ile Ser Thr Ser Pro Gln Pro Pro Thr Thr Lys Thr Gly Pro
    370                 375                 380

Asp Asn Ser Thr His Asn Thr Pro Val Tyr Lys Leu Asp Ile Ser Glu
385                 390                 395                 400

Ala Thr Gln Val Gly Gln His His Arg Arg Ala Asp Asn Asp Ser Thr
                405                 410                 415

Ala Ser Asp Thr Pro Pro Ala Thr Thr Ala Ala Gly Pro Leu Lys Ala
        420                 425                 430

Glu Asn Thr Asn Thr Ser Lys Ser Ala Asp Ser Leu Asp Leu Ala Thr
    435                 440                 445

Thr Thr Ser Pro Gln Asn Tyr Ser Glu Thr Ala Gly Asn Asn Asn Thr
    450                 455                 460

His His Gln Asp Thr Gly Glu Glu Ser Ala Ser Ser Gly Lys Leu Gly
465                 470                 475                 480

Leu Ile Thr Asn Thr Ile Ala Gly Val Ala Gly Leu Ile Thr Gly Gly
                485                 490                 495

Arg Arg Thr Arg Arg Glu Val Ile Val Asn Ala Gln Pro Lys Cys Asn
            500                 505                 510

Pro Asn Leu His Tyr Trp Thr Thr Gln Asp Glu Gly Ala Ala Ile Gly
        515                 520                 525

Leu Ala Trp Ile Pro Tyr Phe Gly Pro Ala Ala Glu Gly Ile Tyr Thr
    530                 535                 540

Glu Gly Leu Met His Asn Gln Asp Gly Leu Ile Cys Gly Leu Arg Gln
545                 550                 555                 560

Leu Ala Asn Glu Thr Thr Gln Ala Leu Gln Leu Phe Leu Arg Ala Thr
                565                 570                 575

Thr Glu Leu Arg Thr Phe Ser Ile Leu Asn Arg Lys Ala Ile Asp Phe
            580                 585                 590

Leu Leu Gln Arg Trp Gly Gly Thr Cys His Ile Leu Gly Pro Asp Cys
        595                 600                 605

Cys Ile Glu Pro His Asp Trp Thr Lys Asn Ile Thr Asp Lys Ile Asp
    610                 615                 620

Gln Ile Ile His Asp Phe Val Asp Lys Thr Leu Pro Asp Gln Gly Asp
625                 630                 635                 640

Asn Asp Asn Trp Trp Thr Gly Trp Arg Gln Trp Ile Pro Ala Gly Ile
                645                 650                 655

Gly Val Thr Gly Val Ile Ile Ala Val Ile Ala Leu Phe Cys Ile Cys
            660                 665                 670

Lys Phe Val Phe
        675

<210>  2
<211>  1347
<212>  DNA
<213>  Artificial Sequence

<220>
<223>  GP 33-311 463-632

<220>
<221>  CDS
<222>  (1)..(1347)

<400>  2
atc ccg ctt gga gtt atc cac aat agt aca tta cag gtt agt gat gtc        48
Ile Pro Leu Gly Val Ile His Asn Ser Thr Leu Gln Val Ser Asp Val
1               5                   10                  15

gac aaa cta gtt tgt cgt gac aaa ctg tca tcc aca aat caa ttg aga        96
Asp Lys Leu Val Cys Arg Asp Lys Leu Ser Ser Thr Asn Gln Leu Arg
            20                  25                  30

tca gtt gga ctg aat ctc gag ggg aat gga gtg gca act gac gtg cca       144
Ser Val Gly Leu Asn Leu Glu Gly Asn Gly Val Ala Thr Asp Val Pro
        35                  40                  45

tct gtg act aaa aga tgg ggc ttc agg tcc ggt gtc cca cca aag gtg       192
Ser Val Thr Lys Arg Trp Gly Phe Arg Ser Gly Val Pro Pro Lys Val
    50                  55                  60

gtc aat tat gaa gct ggt gaa tgg gct gaa aac tgc tac aat ctt gaa       240
Val Asn Tyr Glu Ala Gly Glu Trp Ala Glu Asn Cys Tyr Asn Leu Glu
65                  70                  75                  80

atc aaa aaa cct gac ggg agt gag tgt cta cca gca gcg cca gac ggg       288
Ile Lys Lys Pro Asp Gly Ser Glu Cys Leu Pro Ala Ala Pro Asp Gly
                85                  90                  95

att cgg ggc ttc ccc cgg tgc cgg tat gtg cac aaa gta tca gga acg       336
Ile Arg Gly Phe Pro Arg Cys Arg Tyr Val His Lys Val Ser Gly Thr
            100                 105                 110

gga cca tgt gcc gga gac ttt gcc ttc cac aaa gag ggt gct ttc ttc       384
Gly Pro Cys Ala Gly Asp Phe Ala Phe His Lys Glu Gly Ala Phe Phe
        115                 120                 125

ctg tat gat cga ctt gct tcc aca gtt atc tac cga gga acg act ttc       432
Leu Tyr Asp Arg Leu Ala Ser Thr Val Ile Tyr Arg Gly Thr Thr Phe
    130                 135                 140

gct gaa ggt gtc gtt gca ttt ctg ata ctg ccc caa gct aag aag gac       480
Ala Glu Gly Val Val Ala Phe Leu Ile Leu Pro Gln Ala Lys Lys Asp
145                 150                 155                 160

ttc ttc agc tca cac ccc ttg aga gag ccg gtc aat gca acg gag gac       528
Phe Phe Ser Ser His Pro Leu Arg Glu Pro Val Asn Ala Thr Glu Asp
                165                 170                 175

ccg tcg agt ggc tat tat tct acc aca att aga tat cag gct acc ggt 576
Pro Ser Ser Gly Tyr Tyr Ser Thr Thr Ile Arg Tyr Gln Ala Thr Gly
            180                 185                 190

ttt gga act aat gag aca gag tac ttg ttc gag gtt gac aat ttg acc 624
Phe Gly Thr Asn Glu Thr Glu Tyr Leu Phe Glu Val Asp Asn Leu Thr
        195                 200                 205

tac gtc caa ctt gaa tca aga ttc aca cca cag ttt ctg ctc cag ctg 672
Tyr Val Gln Leu Glu Ser Arg Phe Thr Pro Gln Phe Leu Leu Gln Leu
    210                 215                 220

aat gag aca ata tat gca agt ggg aag agg agc aac acc acg gga aaa 720
Asn Glu Thr Ile Tyr Ala Ser Gly Lys Arg Ser Asn Thr Thr Gly Lys
225                 230                 235                 240

cta att tgg aag gtc aac ccc gaa att gat aca aca atc ggg gag tgg 768
Leu Ile Trp Lys Val Asn Pro Glu Ile Asp Thr Thr Ile Gly Glu Trp
                245                 250                 255

gcc ttc tgg gaa act aaa aaa aac ctc act aga aaa att cgc agt gaa 816
Ala Phe Trp Glu Thr Lys Lys Asn Leu Thr Arg Lys Ile Arg Ser Glu
            260                 265                 270

gag ttg tct ttc aca gct gta aac act cat cac caa gat acc gga gaa 864
Glu Leu Ser Phe Thr Ala Val Asn Thr His His Gln Asp Thr Gly Glu
        275                 280                 285

gag agt gcc agc agc ggg aag cta ggc tta att acc aat act att gct 912
Glu Ser Ala Ser Ser Gly Lys Leu Gly Leu Ile Thr Asn Thr Ile Ala
    290                 295                 300

gga gta gca gga ctg atc aca ggc ggg aga agg act cga aga gaa gta 960
Gly Val Ala Gly Leu Ile Thr Gly Gly Arg Arg Thr Arg Arg Glu Val
305                 310                 315                 320

att gtc aat gct caa ccc aaa tgc aac ccc aat tta cat tac tgg act 1008
Ile Val Asn Ala Gln Pro Lys Cys Asn Pro Asn Leu His Tyr Trp Thr
                325                 330                 335

act cag gat gaa ggt gct gca atc gga ttg gcc tgg ata cca tat ttc 1056
Thr Gln Asp Glu Gly Ala Ala Ile Gly Leu Ala Trp Ile Pro Tyr Phe
            340                 345                 350

ggg cca gca gcc gaa gga att tac aca gag ggg cta atg cac aac caa 1104
Gly Pro Ala Ala Glu Gly Ile Tyr Thr Glu Gly Leu Met His Asn Gln
        355                 360                 365

gat ggt tta atc tgt ggg ttg agg cag ctg gcc aac gaa acg act caa 1152
Asp Gly Leu Ile Cys Gly Leu Arg Gln Leu Ala Asn Glu Thr Thr Gln
    370                 375                 380

gct ctc caa ctg ttc ctg aga gcc aca act gag ctg cga acc ttt tca 1200
Ala Leu Gln Leu Phe Leu Arg Ala Thr Thr Glu Leu Arg Thr Phe Ser
385                 390                 395                 400

atc ctc aac cgt aag gca att gac ttc ctg ctg cag cga tgg ggt ggc 1248
Ile Leu Asn Arg Lys Ala Ile Asp Phe Leu Leu Gln Arg Trp Gly Gly
                405                 410                 415

aca tgc cac att ttg gga ccg gac tgc tgt atc gaa cca cat gat tgg 1296
Thr Cys His Ile Leu Gly Pro Asp Cys Cys Ile Glu Pro His Asp Trp

420                 425                 430

acc aag aac ata aca gac aaa att gat cag att att cat gat ttt gtt      1344
Thr Lys Asn Ile Thr Asp Lys Ile Asp Gln Ile Ile His Asp Phe Val
        435                 440                 445

gat                                                                  1347
Asp

<210>  3
<211>  449
<212>  PRT
<213>  Artificial Sequence

<220>
<223>  Synthetic Construct

<400>  3

Ile Pro Leu Gly Val Ile His Asn Ser Thr Leu Gln Val Ser Asp Val
1               5                   10                  15

Asp Lys Leu Val Cys Arg Asp Lys Leu Ser Ser Thr Asn Gln Leu Arg
            20                  25                  30

Ser Val Gly Leu Asn Leu Glu Gly Asn Gly Val Ala Thr Asp Val Pro
        35                  40                  45

Ser Val Thr Lys Arg Trp Gly Phe Arg Ser Gly Val Pro Pro Lys Val
    50                  55                  60

Val Asn Tyr Glu Ala Gly Glu Trp Ala Glu Asn Cys Tyr Asn Leu Glu
65                  70                  75                  80

Ile Lys Lys Pro Asp Gly Ser Glu Cys Leu Pro Ala Ala Pro Asp Gly
                85                  90                  95

Ile Arg Gly Phe Pro Arg Cys Arg Tyr Val His Lys Val Ser Gly Thr
            100                 105                 110

Gly Pro Cys Ala Gly Asp Phe Ala Phe His Lys Glu Gly Ala Phe Phe
        115                 120                 125

Leu Tyr Asp Arg Leu Ala Ser Thr Val Ile Tyr Arg Gly Thr Thr Phe
    130                 135                 140

Ala Glu Gly Val Val Ala Phe Leu Ile Leu Pro Gln Ala Lys Lys Asp
145                 150                 155                 160

Phe Phe Ser Ser His Pro Leu Arg Glu Pro Val Asn Ala Thr Glu Asp
                165                 170                 175

Pro Ser Ser Gly Tyr Tyr Ser Thr Thr Ile Arg Tyr Gln Ala Thr Gly
            180                 185                 190

Phe Gly Thr Asn Glu Thr Glu Tyr Leu Phe Glu Val Asp Asn Leu Thr
        195                 200                 205

Tyr Val Gln Leu Glu Ser Arg Phe Thr Pro Gln Phe Leu Leu Gln Leu
    210                 215                 220

Asn Glu Thr Ile Tyr Ala Ser Gly Lys Arg Ser Asn Thr Thr Gly Lys
225                 230                 235                 240

Leu Ile Trp Lys Val Asn Pro Glu Ile Asp Thr Thr Ile Gly Glu Trp
                245                 250                 255

Ala Phe Trp Glu Thr Lys Lys Asn Leu Thr Arg Lys Ile Arg Ser Glu
            260                 265                 270

Glu Leu Ser Phe Thr Ala Val Asn Thr His His Gln Asp Thr Gly Glu
        275                 280                 285

Glu Ser Ala Ser Ser Gly Lys Leu Gly Leu Ile Thr Asn Thr Ile Ala
    290                 295                 300

Gly Val Ala Gly Leu Ile Thr Gly Gly Arg Arg Thr Arg Arg Glu Val
305                 310                 315                 320

Ile Val Asn Ala Gln Pro Lys Cys Asn Pro Asn Leu His Tyr Trp Thr
                325                 330                 335

Thr Gln Asp Glu Gly Ala Ala Ile Gly Leu Ala Trp Ile Pro Tyr Phe
            340                 345                 350

Gly Pro Ala Ala Glu Gly Ile Tyr Thr Glu Gly Leu Met His Asn Gln
        355                 360                 365

Asp Gly Leu Ile Cys Gly Leu Arg Gln Leu Ala Asn Glu Thr Thr Gln
    370                 375                 380

Ala Leu Gln Leu Phe Leu Arg Ala Thr Thr Glu Leu Arg Thr Phe Ser
385                 390                 395                 400

Ile Leu Asn Arg Lys Ala Ile Asp Phe Leu Leu Gln Arg Trp Gly Gly
                405                 410                 415

Thr Cys His Ile Leu Gly Pro Asp Cys Cys Ile Glu Pro His Asp Trp

420                 425                 430

Thr Lys Asn Ile Thr Asp Lys Ile Asp Gln Ile Ile His Asp Phe Val
        435                 440                 445

Asp

<210>  4
<211>  1452
<212>  DNA
<213>  Artificial Sequence

<220>
<223>  GP 33-311 463-632 + spacer + Twin strep tag

<220>
<221>  CDS
<222>  (1)..(1452)

<400>  4
atc ccg ctt gga gtt atc cac aat agt aca tta cag gtt agt gat gtc      48
Ile Pro Leu Gly Val Ile His Asn Ser Thr Leu Gln Val Ser Asp Val
1               5                   10                  15

gac aaa cta gtt tgt cgt gac aaa ctg tca tcc aca aat caa ttg aga      96
Asp Lys Leu Val Cys Arg Asp Lys Leu Ser Ser Thr Asn Gln Leu Arg
            20                  25                  30

tca gtt gga ctg aat ctc gag ggg aat gga gtg gca act gac gtg cca     144
Ser Val Gly Leu Asn Leu Glu Gly Asn Gly Val Ala Thr Asp Val Pro
        35                  40                  45

tct gtg act aaa aga tgg ggc ttc agg tcc ggt gtc cca cca aag gtg     192
Ser Val Thr Lys Arg Trp Gly Phe Arg Ser Gly Val Pro Pro Lys Val
    50                  55                  60

gtc aat tat gaa gct ggt gaa tgg gct gaa aac tgc tac aat ctt gaa     240
Val Asn Tyr Glu Ala Gly Glu Trp Ala Glu Asn Cys Tyr Asn Leu Glu
65                  70                  75                  80

atc aaa aaa cct gac ggg agt gag tgt cta cca gca gcg cca gac ggg     288
Ile Lys Lys Pro Asp Gly Ser Glu Cys Leu Pro Ala Ala Pro Asp Gly
                85                  90                  95

att cgg ggc ttc ccc cgg tgc cgg tat gtg cac aaa gta tca gga acg     336
Ile Arg Gly Phe Pro Arg Cys Arg Tyr Val His Lys Val Ser Gly Thr
            100                 105                 110

gga cca tgt gcc gga gac ttt gcc ttc cac aaa gag ggt gct ttc ttc     384
Gly Pro Cys Ala Gly Asp Phe Ala Phe His Lys Glu Gly Ala Phe Phe
        115                 120                 125

ctg tat gat cga ctt gct tcc aca gtt atc tac cga gga acg act ttc     432
Leu Tyr Asp Arg Leu Ala Ser Thr Val Ile Tyr Arg Gly Thr Thr Phe
    130                 135                 140

gct gaa ggt gtc gtt gca ttt ctg ata ctg ccc caa gct aag aag gac     480
Ala Glu Gly Val Val Ala Phe Leu Ile Leu Pro Gln Ala Lys Lys Asp

145                 150                 155                 160

ttc ttc agc tca cac ccc ttg aga gag ccg gtc aat gca acg gag gac       528
Phe Phe Ser Ser His Pro Leu Arg Glu Pro Val Asn Ala Thr Glu Asp
                165                 170                 175

ccg tcg agt ggc tat tat tct acc aca att aga tat cag gct acc ggt       576
Pro Ser Ser Gly Tyr Tyr Ser Thr Thr Ile Arg Tyr Gln Ala Thr Gly
            180                 185                 190

ttt gga act aat gag aca gag tac ttg ttc gag gtt gac aat ttg acc       624
Phe Gly Thr Asn Glu Thr Glu Tyr Leu Phe Glu Val Asp Asn Leu Thr
        195                 200                 205

tac gtc caa ctt gaa tca aga ttc aca cca cag ttt ctg ctc cag ctg       672
Tyr Val Gln Leu Glu Ser Arg Phe Thr Pro Gln Phe Leu Leu Gln Leu
    210                 215                 220

aat gag aca ata tat gca agt ggg aag agg agc aac acc acg gga aaa       720
Asn Glu Thr Ile Tyr Ala Ser Gly Lys Arg Ser Asn Thr Thr Gly Lys
225                 230                 235                 240

cta att tgg aag gtc aac ccc gaa att gat aca aca atc ggg gag tgg       768
Leu Ile Trp Lys Val Asn Pro Glu Ile Asp Thr Thr Ile Gly Glu Trp
                245                 250                 255

gcc ttc tgg gaa act aaa aaa aac ctc act aga aaa att cgc agt gaa       816
Ala Phe Trp Glu Thr Lys Lys Asn Leu Thr Arg Lys Ile Arg Ser Glu
            260                 265                 270

gag ttg tct ttc aca gct gta aac act cat cac caa gat acc gga gaa       864
Glu Leu Ser Phe Thr Ala Val Asn Thr His His Gln Asp Thr Gly Glu
        275                 280                 285

gag agt gcc agc agc ggg aag cta ggc tta att acc aat act att gct       912
Glu Ser Ala Ser Ser Gly Lys Leu Gly Leu Ile Thr Asn Thr Ile Ala
    290                 295                 300

gga gta gca gga ctg atc aca ggc ggg aga agg act cga aga gaa gta       960
Gly Val Ala Gly Leu Ile Thr Gly Gly Arg Arg Thr Arg Arg Glu Val
305                 310                 315                 320

att gtc aat gct caa ccc aaa tgc aac ccc aat tta cat tac tgg act      1008
Ile Val Asn Ala Gln Pro Lys Cys Asn Pro Asn Leu His Tyr Trp Thr
                325                 330                 335

act cag gat gaa ggt gct gca atc gga ttg gcc tgg ata cca tat ttc      1056
Thr Gln Asp Glu Gly Ala Ala Ile Gly Leu Ala Trp Ile Pro Tyr Phe
            340                 345                 350

ggg cca gca gcc gaa gga att tac aca gag ggg cta atg cac aac caa      1104
Gly Pro Ala Ala Glu Gly Ile Tyr Thr Glu Gly Leu Met His Asn Gln
        355                 360                 365

gat ggt tta atc tgt ggg ttg agg cag ctg gcc aac gaa acg act caa      1152
Asp Gly Leu Ile Cys Gly Leu Arg Gln Leu Ala Asn Glu Thr Thr Gln
    370                 375                 380

gct ctc caa ctg ttc ctg aga gcc aca act gag ctg cga acc ttt tca      1200
Ala Leu Gln Leu Phe Leu Arg Ala Thr Thr Glu Leu Arg Thr Phe Ser
385                 390                 395                 400

atc ctc aac cgt aag gca att gac ttc ctg ctg cag cga tgg ggt ggc      1248

Ile Leu Asn Arg Lys Ala Ile Asp Phe Leu Leu Gln Arg Trp Gly Gly
                405                 410                 415

aca tgc cac att ttg gga ccg gac tgc tgt atc gaa cca cat gat tgg      1296
Thr Cys His Ile Leu Gly Pro Asp Cys Cys Ile Glu Pro His Asp Trp
            420                 425                 430

acc aag aac ata aca gac aaa att gat cag att att cat gat ttt gtt      1344
Thr Lys Asn Ile Thr Asp Lys Ile Asp Gln Ile Ile His Asp Phe Val
        435                 440                 445

gat gac gac gat gat aag tgg agc cac ccg cag ttc gaa aaa gga ggc      1392
Asp Asp Asp Asp Asp Lys Trp Ser His Pro Gln Phe Glu Lys Gly Gly
    450                 455                 460

ggt agt gga ggc ggt agt gga ggc agt agt gcc tgg agc cac ccg cag      1440
Gly Ser Gly Gly Gly Ser Gly Gly Ser Ser Ala Trp Ser His Pro Gln
465                 470                 475                 480

ttc gaa aaa taa                                                      1452
Phe Glu Lys

<210>  5
<211>  483
<212>  PRT
<213>  Artificial Sequence

<220>
<223>  Synthetic Construct

<400>  5

Ile Pro Leu Gly Val Ile His Asn Ser Thr Leu Gln Val Ser Asp Val
1               5                   10                  15

Asp Lys Leu Val Cys Arg Asp Lys Leu Ser Ser Thr Asn Gln Leu Arg
            20                  25                  30

Ser Val Gly Leu Asn Leu Glu Gly Asn Gly Val Ala Thr Asp Val Pro
        35                  40                  45

Ser Val Thr Lys Arg Trp Gly Phe Arg Ser Gly Val Pro Pro Lys Val
    50                  55                  60

Val Asn Tyr Glu Ala Gly Glu Trp Ala Glu Asn Cys Tyr Asn Leu Glu
65                  70                  75                  80

Ile Lys Lys Pro Asp Gly Ser Glu Cys Leu Pro Ala Ala Pro Asp Gly
                85                  90                  95

Ile Arg Gly Phe Pro Arg Cys Arg Tyr Val His Lys Val Ser Gly Thr
            100                 105                 110

Gly Pro Cys Ala Gly Asp Phe Ala Phe His Lys Glu Gly Ala Phe Phe

115                 120                 125

Leu Tyr Asp Arg Leu Ala Ser Thr Val Ile Tyr Arg Gly Thr Thr Phe
    130                 135                 140

Ala Glu Gly Val Val Ala Phe Leu Ile Leu Pro Gln Ala Lys Lys Asp
145                 150                 155                 160

Phe Phe Ser Ser His Pro Leu Arg Glu Pro Val Asn Ala Thr Glu Asp
                165                 170                 175

Pro Ser Ser Gly Tyr Tyr Ser Thr Thr Ile Arg Tyr Gln Ala Thr Gly
            180                 185                 190

Phe Gly Thr Asn Glu Thr Glu Tyr Leu Phe Glu Val Asp Asn Leu Thr
        195                 200                 205

Tyr Val Gln Leu Glu Ser Arg Phe Thr Pro Gln Phe Leu Leu Gln Leu
    210                 215                 220

Asn Glu Thr Ile Tyr Ala Ser Gly Lys Arg Ser Asn Thr Thr Gly Lys
225                 230                 235                 240

Leu Ile Trp Lys Val Asn Pro Glu Ile Asp Thr Thr Ile Gly Glu Trp
                245                 250                 255

Ala Phe Trp Glu Thr Lys Lys Asn Leu Thr Arg Lys Ile Arg Ser Glu
            260                 265                 270

Glu Leu Ser Phe Thr Ala Val Asn Thr His His Gln Asp Thr Gly Glu
        275                 280                 285

Glu Ser Ala Ser Ser Gly Lys Leu Gly Leu Ile Thr Asn Thr Ile Ala
    290                 295                 300

Gly Val Ala Gly Leu Ile Thr Gly Gly Arg Arg Thr Arg Arg Glu Val
305                 310                 315                 320

Ile Val Asn Ala Gln Pro Lys Cys Asn Pro Asn Leu His Tyr Trp Thr
                325                 330                 335

Thr Gln Asp Glu Gly Ala Ala Ile Gly Leu Ala Trp Ile Pro Tyr Phe
            340                 345                 350

Gly Pro Ala Ala Glu Gly Ile Tyr Thr Glu Gly Leu Met His Asn Gln
        355                 360                 365

Asp Gly Leu Ile Cys Gly Leu Arg Gln Leu Ala Asn Glu Thr Thr Gln
    370                 375                 380

Ala Leu Gln Leu Phe Leu Arg Ala Thr Thr Glu Leu Arg Thr Phe Ser
385                 390                 395                 400

Ile Leu Asn Arg Lys Ala Ile Asp Phe Leu Leu Gln Arg Trp Gly Gly
                405                 410                 415

Thr Cys His Ile Leu Gly Pro Asp Cys Cys Ile Glu Pro His Asp Trp
            420                 425                 430

Thr Lys Asn Ile Thr Asp Lys Ile Asp Gln Ile Ile His Asp Phe Val
        435                 440                 445

Asp Asp Asp Asp Asp Lys Trp Ser His Pro Gln Phe Glu Lys Gly Gly
    450                 455                 460

Gly Ser Gly Gly Gly Ser Gly Gly Ser Ser Ala Trp Ser His Pro Gln
465                 470                 475                 480

Phe Glu Lys

<210>  6
<211>  1392
<212>  DNA
<213>  Artificial Sequence

<220>
<223>  GP 33-311 463-632 + spacer + His tag

<220>
<221>  CDS
<222>  (1)..(1392)

<400>  6
atc ccg ctt gga gtt atc cac aat agt aca tta cag gtt agt gat gtc      48
Ile Pro Leu Gly Val Ile His Asn Ser Thr Leu Gln Val Ser Asp Val
1               5                   10                  15

gac aaa cta gtt tgt cgt gac aaa ctg tca tcc aca aat caa ttg aga      96
Asp Lys Leu Val Cys Arg Asp Lys Leu Ser Ser Thr Asn Gln Leu Arg
            20                  25                  30

tca gtt gga ctg aat ctc gag ggg aat gga gtg gca act gac gtg cca      144
Ser Val Gly Leu Asn Leu Glu Gly Asn Gly Val Ala Thr Asp Val Pro
        35                  40                  45

tct gtg act aaa aga tgg ggc ttc agg tcc ggt gtc cca cca aag gtg      192
Ser Val Thr Lys Arg Trp Gly Phe Arg Ser Gly Val Pro Pro Lys Val
    50                  55                  60

gtc aat tat gaa gct ggt gaa tgg gct gaa aac tgc tac aat ctt gaa      240

Val Asn Tyr Glu Ala Gly Glu Trp Ala Glu Asn Cys Tyr Asn Leu Glu
65                  70                  75                  80

atc aaa aaa cct gac ggg agt gag tgt cta cca gca gcg cca gac ggg      288
Ile Lys Lys Pro Asp Gly Ser Glu Cys Leu Pro Ala Ala Pro Asp Gly
                85                  90                  95

att cgg ggc ttc ccc cgg tgc cgg tat gtg cac aaa gta tca gga acg      336
Ile Arg Gly Phe Pro Arg Cys Arg Tyr Val His Lys Val Ser Gly Thr
            100                 105                 110

gga cca tgt gcc gga gac ttt gcc ttc cac aaa gag ggt gct ttc ttc      384
Gly Pro Cys Ala Gly Asp Phe Ala Phe His Lys Glu Gly Ala Phe Phe
        115                 120                 125

ctg tat gat cga ctt gct tcc aca gtt atc tac cga gga acg act ttc      432
Leu Tyr Asp Arg Leu Ala Ser Thr Val Ile Tyr Arg Gly Thr Thr Phe
    130                 135                 140

gct gaa ggt gtc gtt gca ttt ctg ata ctg ccc caa gct aag aag gac      480
Ala Glu Gly Val Val Ala Phe Leu Ile Leu Pro Gln Ala Lys Lys Asp
145                 150                 155                 160

ttc ttc agc tca cac ccc ttg aga gag ccg gtc aat gca acg gag gac      528
Phe Phe Ser Ser His Pro Leu Arg Glu Pro Val Asn Ala Thr Glu Asp
                165                 170                 175

ccg tcg agt ggc tat tat tct acc aca att aga tat cag gct acc ggt      576
Pro Ser Ser Gly Tyr Tyr Ser Thr Thr Ile Arg Tyr Gln Ala Thr Gly
            180                 185                 190

ttt gga act aat gag aca gag tac ttg ttc gag gtt gac aat ttg acc      624
Phe Gly Thr Asn Glu Thr Glu Tyr Leu Phe Glu Val Asp Asn Leu Thr
        195                 200                 205

tac gtc caa ctt gaa tca aga ttc aca cca cag ttt ctg ctc cag ctg      672
Tyr Val Gln Leu Glu Ser Arg Phe Thr Pro Gln Phe Leu Leu Gln Leu
    210                 215                 220

aat gag aca ata tat gca agt ggg aag agg agc aac acc acg gga aaa      720
Asn Glu Thr Ile Tyr Ala Ser Gly Lys Arg Ser Asn Thr Thr Gly Lys
225                 230                 235                 240

cta att tgg aag gtc aac ccc gaa att gat aca aca atc ggg gag tgg      768
Leu Ile Trp Lys Val Asn Pro Glu Ile Asp Thr Thr Ile Gly Glu Trp
                245                 250                 255

gcc ttc tgg gaa act aaa aaa aac ctc act aga aaa att cgc agt gaa      816
Ala Phe Trp Glu Thr Lys Lys Asn Leu Thr Arg Lys Ile Arg Ser Glu
            260                 265                 270

gag ttg tct ttc aca gct gta aac act cat cac caa gat acc gga gaa      864
Glu Leu Ser Phe Thr Ala Val Asn Thr His His Gln Asp Thr Gly Glu
        275                 280                 285

gag agt gcc agc agc ggg aag cta ggc tta att acc aat act att gct      912
Glu Ser Ala Ser Ser Gly Lys Leu Gly Leu Ile Thr Asn Thr Ile Ala
    290                 295                 300

gga gta gca gga ctg atc aca ggc ggg aga agg act cga aga gaa gta      960
Gly Val Ala Gly Leu Ile Thr Gly Gly Arg Arg Thr Arg Arg Glu Val
305                 310                 315                 320

att gtc aat gct caa ccc aaa tgc aac ccc aat tta cat tac tgg act 1008
Ile Val Asn Ala Gln Pro Lys Cys Asn Pro Asn Leu His Tyr Trp Thr
                325                 330                 335

act cag gat gaa ggt gct gca atc gga ttg gcc tgg ata cca tat ttc 1056
Thr Gln Asp Glu Gly Ala Ala Ile Gly Leu Ala Trp Ile Pro Tyr Phe
            340                 345                 350

ggg cca gca gcc gaa gga att tac aca gag ggg cta atg cac aac caa 1104
Gly Pro Ala Ala Glu Gly Ile Tyr Thr Glu Gly Leu Met His Asn Gln
        355                 360                 365

gat ggt tta atc tgt ggg ttg agg cag ctg gcc aac gaa acg act caa 1152
Asp Gly Leu Ile Cys Gly Leu Arg Gln Leu Ala Asn Glu Thr Thr Gln
    370                 375                 380

gct ctc caa ctg ttc ctg aga gcc aca act gag ctg cga acc ttt tca 1200
Ala Leu Gln Leu Phe Leu Arg Ala Thr Thr Glu Leu Arg Thr Phe Ser
385                 390                 395                 400

atc ctc aac cgt aag gca att gac ttc ctg ctg cag cga tgg ggt ggc 1248
Ile Leu Asn Arg Lys Ala Ile Asp Phe Leu Leu Gln Arg Trp Gly Gly
                405                 410                 415

aca tgc cac att ttg gga ccg gac tgc tgt atc gaa cca cat gat tgg 1296
Thr Cys His Ile Leu Gly Pro Asp Cys Cys Ile Glu Pro His Asp Trp
            420                 425                 430

acc aag aac ata aca gac aaa att gat cag att att cat gat ttt gtt 1344
Thr Lys Asn Ile Thr Asp Lys Ile Asp Gln Ile Ile His Asp Phe Val
        435                 440                 445

gat gac gac gat gat aag cat cat cac cat cac cat cac cat cac cat 1392
Asp Asp Asp Asp Asp Lys His His His His His His His His His His
    450                 455                 460

<210> 7
<211> 464
<212> PRT
<213> Artificial Sequence

<220>
<223> Synthetic Construct

<400> 7

Ile Pro Leu Gly Val Ile His Asn Ser Thr Leu Gln Val Ser Asp Val
1               5                   10                  15

Asp Lys Leu Val Cys Arg Asp Lys Leu Ser Ser Thr Asn Gln Leu Arg
            20                  25                  30

Ser Val Gly Leu Asn Leu Glu Gly Asn Gly Val Ala Thr Asp Val Pro
        35                  40                  45

Ser Val Thr Lys Arg Trp Gly Phe Arg Ser Gly Val Pro Pro Lys Val
    50                  55                  60

Val Asn Tyr Glu Ala Gly Glu Trp Ala Glu Asn Cys Tyr Asn Leu Glu
65                  70                  75                  80

Ile Lys Lys Pro Asp Gly Ser Glu Cys Leu Pro Ala Ala Pro Asp Gly
                85                  90                  95

Ile Arg Gly Phe Pro Arg Cys Arg Tyr Val His Lys Val Ser Gly Thr
            100                 105                 110

Gly Pro Cys Ala Gly Asp Phe Ala Phe His Lys Glu Gly Ala Phe Phe
        115                 120                 125

Leu Tyr Asp Arg Leu Ala Ser Thr Val Ile Tyr Arg Gly Thr Thr Phe
    130                 135                 140

Ala Glu Gly Val Val Ala Phe Leu Ile Leu Pro Gln Ala Lys Lys Asp
145                 150                 155                 160

Phe Phe Ser Ser His Pro Leu Arg Glu Pro Val Asn Ala Thr Glu Asp
                165                 170                 175

Pro Ser Ser Gly Tyr Tyr Ser Thr Thr Ile Arg Tyr Gln Ala Thr Gly
            180                 185                 190

Phe Gly Thr Asn Glu Thr Glu Tyr Leu Phe Glu Val Asp Asn Leu Thr
        195                 200                 205

Tyr Val Gln Leu Glu Ser Arg Phe Thr Pro Gln Phe Leu Leu Gln Leu
    210                 215                 220

Asn Glu Thr Ile Tyr Ala Ser Gly Lys Arg Ser Asn Thr Thr Gly Lys
225                 230                 235                 240

Leu Ile Trp Lys Val Asn Pro Glu Ile Asp Thr Thr Ile Gly Glu Trp
                245                 250                 255

Ala Phe Trp Glu Thr Lys Lys Asn Leu Thr Arg Lys Ile Arg Ser Glu
            260                 265                 270

Glu Leu Ser Phe Thr Ala Val Asn Thr His His Gln Asp Thr Gly Glu
        275                 280                 285

Glu Ser Ala Ser Ser Gly Lys Leu Gly Leu Ile Thr Asn Thr Ile Ala
    290                 295                 300

Gly Val Ala Gly Leu Ile Thr Gly Gly Arg Arg Thr Arg Arg Glu Val
305                 310                 315                 320

Ile Val Asn Ala Gln Pro Lys Cys Asn Pro Asn Leu His Tyr Trp Thr
                325                 330                 335

Thr Gln Asp Glu Gly Ala Ala Ile Gly Leu Ala Trp Ile Pro Tyr Phe
            340                 345                 350

Gly Pro Ala Ala Glu Gly Ile Tyr Thr Glu Gly Leu Met His Asn Gln
        355                 360                 365

Asp Gly Leu Ile Cys Gly Leu Arg Gln Leu Ala Asn Glu Thr Thr Gln
    370                 375                 380

Ala Leu Gln Leu Phe Leu Arg Ala Thr Thr Glu Leu Arg Thr Phe Ser
385                 390                 395                 400

Ile Leu Asn Arg Lys Ala Ile Asp Phe Leu Leu Gln Arg Trp Gly Gly
                405                 410                 415

Thr Cys His Ile Leu Gly Pro Asp Cys Cys Ile Glu Pro His Asp Trp
            420                 425                 430

Thr Lys Asn Ile Thr Asp Lys Ile Asp Gln Ile Ile His Asp Phe Val
        435                 440                 445

Asp Asp Asp Asp Asp Lys His His His His His His His His His His
    450                 455                 460

<210>  8
<211>  500
<212>  PRT
<213>  Artificial Sequence

<220>
<223>  GP1 subunit consensus sequence

<400>  8

Met Gly Val Thr Gly Ile Leu Gln Leu Pro Arg Asp Arg Phe Lys Arg
1               5                   10                  15

Thr Ser Phe Phe Leu Trp Val Ile Ile Leu Phe Gln Arg Thr Phe Ser
            20                  25                  30

Ile Pro Leu Gly Val Ile His Asn Ser Thr Leu Gln Val Ser Asp Val
        35                  40                  45

Asp Lys Leu Val Cys Arg Asp Lys Leu Ser Ser Thr Asn Gln Leu Arg
    50                  55                  60

Ser Val Gly Leu Asn Leu Glu Gly Asn Gly Val Ala Thr Asp Val Pro
65                  70                  75                  80

Ser Val Thr Lys Arg Trp Gly Phe Arg Ser Gly Val Pro Pro Lys Val
                85                  90                  95

Val Asn Tyr Glu Ala Gly Glu Trp Ala Glu Asn Cys Tyr Asn Leu Glu
            100                 105                 110

Ile Lys Lys Pro Asp Gly Ser Glu Cys Leu Pro Ala Ala Pro Asp Gly
        115                 120                 125

Ile Arg Gly Phe Pro Arg Cys Arg Tyr Val His Lys Val Ser Gly Thr
    130                 135                 140

Gly Pro Cys Ala Gly Asp Phe Ala Phe His Lys Glu Gly Ala Phe Phe
145                 150                 155                 160

Leu Tyr Asp Arg Leu Ala Ser Thr Val Ile Tyr Arg Gly Thr Thr Phe
                165                 170                 175

Ala Glu Gly Val Val Ala Phe Leu Ile Leu Pro Gln Ala Lys Lys Asp
            180                 185                 190

Phe Phe Ser Ser His Pro Leu Arg Glu Pro Val Asn Ala Thr Glu Asp
        195                 200                 205

Pro Ser Ser Gly Tyr Tyr Ser Thr Thr Ile Arg Tyr Gln Ala Thr Gly
    210                 215                 220

Phe Gly Thr Asn Glu Thr Glu Tyr Leu Phe Glu Val Asp Asn Leu Thr
225                 230                 235                 240

Tyr Val Gln Leu Glu Ser Arg Phe Thr Pro Gln Phe Leu Leu Gln Leu
                245                 250                 255

Asn Glu Thr Ile Tyr Ala Ser Gly Lys Arg Ser Asn Thr Thr Gly Lys
            260                 265                 270

Leu Ile Trp Lys Val Asn Pro Glu Ile Asp Thr Thr Ile Gly Glu Trp
        275                 280                 285

Ala Phe Trp Glu Thr Lys Lys Asn Leu Thr Arg Lys Ile Arg Ser Glu
    290                 295                 300

Glu Leu Ser Phe Thr Ala Val Ser Asn Gly Pro Lys Asn Ile Ser Gly
305                 310                 315                 320

Gln Ser Pro Ala Arg Thr Ser Ser Asp Pro Glu Thr Asn Thr Thr Asn
                325                 330                 335

Glu Asp His Lys Ile Met Ala Ser Glu Asn Ser Ser Ala Met Val Gln
            340                 345                 350

Val His Ser Gln Gly Arg Lys Ala Ala Val Ser His Leu Thr Thr Leu
        355                 360                 365

Ala Thr Ile Ser Thr Ser Pro Gln Pro Pro Thr Thr Lys Thr Gly Pro
    370                 375                 380

Asp Asn Ser Thr His Asn Thr Pro Val Tyr Lys Leu Asp Ile Ser Glu
385                 390                 395                 400

Ala Thr Gln Val Gly Gln His His Arg Arg Ala Asp Asn Asp Ser Thr
                405                 410                 415

Ala Ser Asp Thr Pro Pro Ala Thr Thr Ala Ala Gly Pro Leu Lys Ala
            420                 425                 430

Glu Asn Thr Asn Thr Ser Lys Ser Ala Asp Ser Leu Asp Leu Ala Thr
        435                 440                 445

Thr Thr Ser Pro Gln Asn Tyr Ser Glu Thr Ala Gly Asn Asn Asn Thr
    450                 455                 460

His His Gln Asp Thr Gly Glu Glu Ser Ala Ser Ser Gly Lys Leu Gly
465                 470                 475                 480

Leu Ile Thr Asn Thr Ile Ala Gly Val Ala Gly Leu Ile Thr Gly Gly
                485                 490                 495

Arg Arg Thr Arg
            500

<210>  9
<211>  176
<212>  PRT
<213>  Artificial Sequence

<220>
<223>  GP2 subunit consensus sequence

<400>  9

Arg Glu Val Ile Val Asn Ala Gln Pro Lys Cys Asn Pro Asn Leu His
1               5                   10                  15

Tyr Trp Thr Thr Gln Asp Glu Gly Ala Ala Ile Gly Leu Ala Trp Ile
            20                  25                  30

Pro Tyr Phe Gly Pro Ala Ala Glu Gly Ile Tyr Thr Glu Gly Leu Met
        35                  40                  45

His Asn Gln Asp Gly Leu Ile Cys Gly Leu Arg Gln Leu Ala Asn Glu
    50                  55                  60

Thr Thr Gln Ala Leu Gln Leu Phe Leu Arg Ala Thr Thr Glu Leu Arg
65                  70                  75                  80

Thr Phe Ser Ile Leu Asn Arg Lys Ala Ile Asp Phe Leu Leu Gln Arg
                85                  90                  95

Trp Gly Gly Thr Cys His Ile Leu Gly Pro Asp Cys Cys Ile Glu Pro
            100                 105                 110

His Asp Trp Thr Lys Asn Ile Thr Asp Lys Ile Asp Gln Ile Ile His
        115                 120                 125

Asp Phe Val Asp Lys Thr Leu Pro Asp Gln Gly Asp Asn Asp Asn Trp
    130                 135                 140

Trp Thr Gly Trp Arg Gln Trp Ile Pro Ala Gly Ile Gly Val Thr Gly
145                 150                 155                 160

Val Ile Ile Ala Val Ile Ala Leu Phe Cys Ile Cys Lys Phe Val Phe
                165                 170                 175

<210>  10
<211>  5
<212>  PRT
<213>  Artificial Sequence

<220>
<223>  Enterokinase cleavage site spacer

<400>  10

Asp Asp Asp Asp Lys
1               5

<210>  11
<211>  454
<212>  PRT
<213>  Artificial Sequence

<220>
<223>  GP 33-311 463-632 + spacer

<400>  11

Ile Pro Leu Gly Val Ile His Asn Ser Thr Leu Gln Val Ser Asp Val
1               5                   10                  15

Asp Lys Leu Val Cys Arg Asp Lys Leu Ser Ser Thr Asn Gln Leu Arg
            20                  25                  30

Ser Val Gly Leu Asn Leu Glu Gly Asn Gly Val Ala Thr Asp Val Pro
        35                  40                  45

Ser Val Thr Lys Arg Trp Gly Phe Arg Ser Gly Val Pro Pro Lys Val
    50                  55                  60

Val Asn Tyr Glu Ala Gly Glu Trp Ala Glu Asn Cys Tyr Asn Leu Glu
65                  70                  75                  80

Ile Lys Lys Pro Asp Gly Ser Glu Cys Leu Pro Ala Ala Pro Asp Gly
                85                  90                  95

Ile Arg Gly Phe Pro Arg Cys Arg Tyr Val His Lys Val Ser Gly Thr
            100                 105                 110

Gly Pro Cys Ala Gly Asp Phe Ala Phe His Lys Glu Gly Ala Phe Phe
        115                 120                 125

Leu Tyr Asp Arg Leu Ala Ser Thr Val Ile Tyr Arg Gly Thr Thr Phe
    130                 135                 140

Ala Glu Gly Val Val Ala Phe Leu Ile Leu Pro Gln Ala Lys Lys Asp
145                 150                 155                 160

Phe Phe Ser Ser His Pro Leu Arg Glu Pro Val Asn Ala Thr Glu Asp
                165                 170                 175

Pro Ser Ser Gly Tyr Tyr Ser Thr Thr Ile Arg Tyr Gln Ala Thr Gly
            180                 185                 190

Phe Gly Thr Asn Glu Thr Glu Tyr Leu Phe Glu Val Asp Asn Leu Thr
        195                 200                 205

Tyr Val Gln Leu Glu Ser Arg Phe Thr Pro Gln Phe Leu Leu Gln Leu
    210                 215                 220

Asn Glu Thr Ile Tyr Ala Ser Gly Lys Arg Ser Asn Thr Thr Gly Lys
225                 230                 235                 240

Leu Ile Trp Lys Val Asn Pro Glu Ile Asp Thr Thr Ile Gly Glu Trp
                245                 250                 255

Ala Phe Trp Glu Thr Lys Lys Asn Leu Thr Arg Lys Ile Arg Ser Glu
            260                 265                 270

Glu Leu Ser Phe Thr Ala Val Asn Thr His His Gln Asp Thr Gly Glu
        275                 280                 285

Glu Ser Ala Ser Ser Gly Lys Leu Gly Leu Ile Thr Asn Thr Ile Ala
    290                 295                 300

Gly Val Ala Gly Leu Ile Thr Gly Gly Arg Arg Thr Arg Arg Glu Val
305                 310                 315                 320

Ile Val Asn Ala Gln Pro Lys Cys Asn Pro Asn Leu His Tyr Trp Thr
                325                 330                 335

Thr Gln Asp Glu Gly Ala Ala Ile Gly Leu Ala Trp Ile Pro Tyr Phe
            340                 345                 350

Gly Pro Ala Ala Glu Gly Ile Tyr Thr Glu Gly Leu Met His Asn Gln
        355                 360                 365

Asp Gly Leu Ile Cys Gly Leu Arg Gln Leu Ala Asn Glu Thr Thr Gln
    370                 375                 380

Ala Leu Gln Leu Phe Leu Arg Ala Thr Thr Glu Leu Arg Thr Phe Ser
385                 390                 395                 400

Ile Leu Asn Arg Lys Ala Ile Asp Phe Leu Leu Gln Arg Trp Gly Gly
                405                 410                 415

Thr Cys His Ile Leu Gly Pro Asp Cys Cys Ile Glu Pro His Asp Trp
            420                 425                 430

Thr Lys Asn Ile Thr Asp Lys Ile Asp Gln Ile Ile His Asp Phe Val
        435                 440                 445

Asp Asp Asp Asp Asp Lys
    450

## Description

### Field of the invention

The present invention relates to the prevention and/or treatment of the Ebola virus disease.

### Background

Ebola virus disease (EVD), also known as Ebola hemorrhagic fever, is a severe and often fatal illness in humans. According to the World Health Organization, the average case fatality rate is indeed around 50% (from 25% to 90% in past outbreaks).

The virus is transmitted to humans from wild animals and then spreads in the human population via direct contact (for example with blood, secretions, other bodily fluids, or organs of infected people) or indirect contact (for example with contaminated surfaces or materials).

Mortality results from multi-organ failure and severe bleeding complications. The incubation period is from 2 to 21 days (usually 4-6 days). The symptoms comprise fever, fatigue, muscle pain, severe headache, sore throat, vomiting, diarrhea, rash, symptoms of impaired kidney and liver function and, in some cases, both internal and external bleeding. The disease is also associated with a decrease in blood cells and platelets, as well as increased liver enzymes.

There is no approved vaccine or drug able to neutralize the virus. The treatment of patients infected with Ebola virus thus essentially consists in rehydration and treatment of the specific symptoms.

Among the promising drug candidates to treat Ebola virus disease are ZMapp (a cocktail of three humanized monoclonal antibodies), TKM-Ebola (a RNA polymerase blocker using RNAi), Favipiravir (a small molecule acting as RNA polymerase inhibitor) and BCX-4430 (adenosine nucleoside analogue). Vaccine candidates include cAD3 Ebola vaccine (a recombinant Chimpanzee adenovirus serotype 3 vectored Ebola vaccine) and VSVΔG-ZEBOV (a recombinant vesicular stomatitis virus-based vaccine).

When considering antibody-based therapy, humanized monoclonal antibodies are used, because of their high specificity, homogeneity and limitless supply.

There is still a need of therapeutic compounds for the prevention and/or treatment of the Ebola disease.

### Description of the invention

The Inventors have surprisingly found that polyclonal antibodies may be used for the prevention and/or treatment of the Ebola virus disease, in particular in humans.

The Inventors have indeed shown that polyclonal antibodies specifically binding to a modified Ebola virus GP glycoprotein are active against the Ebola virus. Said polyclonal antibodies were purified from the serum of rabbits immunized with a truncated Ebola GP glycoprotein comprising sequence SEQ ID NO: 11.

The present invention is thus based on passive immunotherapy. The passive transfer of antibodies confers two possible types of protective immunity: either a complete inhibition of virus replication, leading to the so-called sterile immunity, either an incomplete inhibition of virus replication. In the case of incomplete virus inhibition, the virus replicates at low level, the reduced viral load enabling the host to mount virus induced immune responses leading to the complete elimination of the virus.

One object of the invention thus relates to polyclonal antibodies specific for the Ebola virus.

Polyclonal antibodies present the advantages of being generated rapidly (for example in a few weeks), more easily and at less expense then monoclonal antibodies. Besides, because they recognize multiple epitopes, polyclonal antibodies usually have a broader activity spectrum than monoclonal antibodies.

The polyclonal antibodies are preferably non-human polyclonal antibodies.

The polyclonal antibodies may be selected in the group consisting of rabbit polyclonal antibodies, mice polyclonal antibodies, rat polyclonal antibodies, guinea pig polyclonal antibodies, chicken polyclonal antibodies, goat polyclonal antibodies, horse polyclonal antibodies, cow polyclonal antibodies, sheep polyclonal antibodies and their combinations.

In a preferred embodiment, the polyclonal antibodies specifically bind to the Ebola virus GP glycoprotein, the Ebola virus GP1 subunit, the Ebola virus GP2 subunit and/or a variant thereof, for example a truncated form of an Ebola virus GP glycoprotein, preferably a truncated form derived from the Ebola virus GP glycoprotein of sequence SEQ ID NO: 1.

The polyclonal antibodies preferably specifically bind to a variant of the Ebola virus GP glycoprotein, wherein said variant comprises or consists of a sequence at least 80% identical to sequence SEQ ID NO: 3, SEQ ID NO: 5, SEQ ID NO: 7 and/or SEQ ID NO: 11 and/or wherein said variant is encoded by a nucleic acid comprising or consisting of a sequence at least 80% identical to SEQ ID NO: 2, SEQ ID NO: 4 and/or SEQ ID NO: 6.

The polyclonal antibodies are preferably obtained from at least one biological sample of an animal immunized with (i) at least one antigen as defined above, preferably comprising or consisting of a sequence at least 80% identical to sequence SEQ ID NO: 1, SEQ ID NO: 3, SEQ ID NO: 5, SEQ ID NO: 7, SEQ ID NO: 8, SEQ ID NO: 9 or SEQ ID NO: 11 and/or (ii) at least one nucleic acid comprising or consisting of a sequence encoding said antigen and/or (iii) at least one vector comprising said nucleic acid.

One other object of the invention is a pharmaceutical composition comprising the polyclonal antibodies as defined above.

The pharmaceutical composition is preferably serum-free.

The pharmaceutical composition is preferably suitable for subcutaneous, intradermal, intramuscular, intraperitoneal and/or intravenous administration.

One other object of the invention is a method for producing the polyclonal antibodies as defined above, wherein said method comprises:
- providing at least one biological sample from an animal immunized with:
   (i) at least one antigen selected in the group consisting of the Ebola virus GP glycoprotein, the Ebola virus GP1 subunit, the Ebola virus GP2 subunit and a variant thereof, said antigen preferably comprising or consisting of a sequence at least 80% identical to sequence SEQ ID NO: 1, SEQ ID NO: 3, SEQ ID NO: 5, SEQ ID NO: 7, SEQ ID NO: 8, SEQ ID NO: 9 or SEQ ID NO: 11 and/or
   (ii) at least one nucleic acid comprising or consisting of a sequence encoding said antigen and/or (iii) at least one vector comprising a nucleic acid , said nucleic acid comprising or consisting of a sequence encoding said antigen, and
- purifying the polyclonal antibodies from said biological sample.

One other object of the invention relates to the polyclonal antibodies as defined above or to the pharmaceutical composition as defined above, for use in a method for the prevention and/or treatment of Ebola virus disease.

Said Ebola virus disease is preferably caused by at least one virus selected in the group consisting of *Zaire Ebolavirus*, *Bundibugyo Ebolavirus, Reston Ebolavirus*, *Sudan Ebolavirus* and *Taï Forest Ebolavirus,* preferably caused by *Zaire Ebolavirus.*

The polyclonal antibodies according to the invention may be used in combination with at least another anti-Ebola virus drug and/or at least another anti-Ebola virus vaccine.

One other object of the invention is a method for preventing and/or treating the Ebola virus disease in a subject in need thereof, said method comprising administering polyclonal antibodies as defined above or a pharmaceutical composition as defined above to said subject.

The present invention also relates to an antigen comprising or consisting of a sequence at least 80% identical to sequence SEQ ID NO: 1, SEQ ID NO: 3, SEQ ID NO: 5, SEQ ID NO: 7, SEQ ID NO: 8, SEQ ID NO: 9 or SEQ ID NO: 11, a nucleic acid comprising or consisting of a sequence encoding said antigen and a vector comprising said nucleic acid.

### Ebola virus

The Ebola virus belongs to the *Filoviridae* family and the *Ebolavirus* genus.

The Ebola virus may be of any known Ebola virus species.

Preferably, the Ebola virus may be selected in the group consisting of *Zaire Ebolavirus* (also called ZEBOV or EBOV), *Bundibugyo Ebolavirus* (also called BEBOV or BDBV), *Reston Ebolavirus* (also called REBOV or RESTV), *Sudan Ebolavirus* (also called SEBOV or SUDV) and *Taï Forest Ebolavirus* (also called TAFV, ICEBOV or *Ivory Coast Ebolavirus*).

The Ebola virus is preferably *Zaire Ebolavirus* or *Sudan Ebolavirus*, preferably *Zaire Ebolavirus.*

The Ebola virus consists of a non-segmented, negative single-stranded linear RNA.

Seven proteins are encoded by the Ebola virus genome: the nucleoprotein (also called NP), VP35, VP40, the GP glycoprotein (also called GP or GP protein), VP30, VP24 and the RNA-dependent RNA Polymerase protein (also called L).

NP, VP30, VP35 and L bind to the negative-sense RNA genome and form the ribonucleoprotein complex.

VP40 is the major matrix protein and VP24 is the minor matrix protein.

GP is the major membrane spike protein and is encoded by the GP gene.

As a matter of fact, the GP gene produces two proteins, which are sGP and GP. sGP is a small polypeptide.

GP consists in a N-terminus GP1 subunit and a C-terminal GP2 subunit, both linked by disulfide bonds. GP is therefore also called GP1/GP2 heterodimer.

The GP1 subunit, also called GP1 protein or GP1, consists of a N-terminus half, a head and a C-terminus domain. The N-terminus half of the GP1 subunit is highly conserved. The head of the GP1 subunit comprises the receptor-binding domain. The C-terminus domain of the GP1 subunit comprises two highly glycosylated regions called the glycan cap and the mucin domain.

The GP2 subunit, also called GP2 protein or GP2, comprises a transmembrane domain, a fusion peptide and two heptad repeat (HR) domains that are required for virus-cell membrane fusion.

The mature GP protein exists as homo-trimers on the surface of viral particles, the trimerization being mediated by GP2.

### Ebola virus antigen

As used herein, the term "antigen" refers to any substance that, when recognized as non-self by the adaptive immune system of an animal (human or non-human), triggers an immune response.

At least one Ebola virus antigen is preferably used to obtain the polyclonal antibodies according to the present invention.

The Ebola virus antigen is preferably selected in the group consisting of the Ebola virus GP glycoprotein, the Ebola virus GP1 subunit, the Ebola virus GP2 subunit and/or a variant thereof.

Many sequences of naturally occurring Ebola virus GP glycoproteins and their subunits GP1 and GP2 are known from public gene databases.

The Inventors have developed a consensus sequence for the Ebola virus GP glycoprotein from 899 sequences of the Zaire Ebola virus GP glycoprotein. This consensus sequence of 676 amino acids is referred to as sequence SEQ ID NO: 1. Although being a consensus sequence, sequence SEQ ID NO: 1 is herein considered as an Ebola virus GP glycoprotein sequence.

The Ebola virus GP glycoprotein may thus comprise or consist of a sequence at least 80% identical, preferably at least 85% identical, more preferably at least 90% identical, still more preferably at least 95%, 96%, 97%, 98% or 99% identical to sequence SEQ ID NO: 1. The Ebola virus GP glycoprotein preferably comprises or consists of sequence SEQ ID NO: 1.

A reference sequence for the Ebola virus GP1 subunit is sequence SEQ ID NO: 8, which corresponds to amino acids 1 to 500 of sequence SEQ ID NO: 1.

The Ebola virus GP1 subunit may comprise or consist of a sequence at least 80% identical, preferably at least 85% identical, more preferably at least 90% identical, still more preferably at least 95%, 96%, 97%, 98% or 99% identical to sequence SEQ ID NO: 8. The Ebola virus GP1 subunit preferably comprises or consists of sequence SEQ ID NO: 8.

A reference sequence for the Ebola virus GP2 subunit is sequence SEQ ID NO: 9, which corresponds to amino acids 501 to 676 of sequence SEQ ID NO: 1.

The Ebola virus GP2 subunit may comprise or consist of a sequence at least 80% identical, preferably at least 85% identical, more preferably at least 90% identical, still more preferably at least 95%, 96%, 97%, 98% or 99% identical to sequence SEQ ID NO: 9. The Ebola virus GP2 subunit preferably comprises or consists of sequence SEQ ID NO: 9.

The expression "variant of a given protein" herein means a derivative of said protein, wherein said derivative shares completely or at least partially the immunogenic properties of said protein. A variant of a given protein preferably comprises at least one epitope of said protein.

A variant of a given protein is for example a biologically and/or chemically modified form of said protein, such as a fragment, a truncated form and/or a soluble form of said protein, wherein said fragment and/or truncated form and/or soluble form may comprise additional biological and/or chemical modification(s).

The biological and/or chemical modifications may increase the immunogenicity, the stability and/or the time of circulation before clearance of the variant by comparison to the protein from which it derives and/or may facilitate the purification of said variant.

Using a soluble form of a protein as an antigen is particularly advantageous for a membrane protein and/or to make the production of the antigen easier.

To facilitate purification, the variant may comprise at least one tag.

Tags for the purification of proteins are well known by the skilled person. Non-limitative examples of tag are a Strep-tag or a Histidine tag.

When present, a tag is preferably linked directely or indirectly (for example via a linker) to the C-terminal part of the protein.

A spacer may advantageously be added to the C-terminal part of the protein, for example between the protein and the at least one tag.

The spacer may be a cleavage site spacer, preferably an enzymatic cleavage site spacer, such as an enterokinase cleavage site spacer. An example of enterokinase cleavage site spacer is DDDDK (SEQ ID NO: 10).

The variant may also comprise amino acids resulting from the cleavage of a cleavage site spacer.

A preferred variant of a GP1 subunit comprises or consists of (i) at least one fragment, for example one or two fragments, of said GP1 subunit, wherein said fragment preferably lacks at least one part of the mucin domain or at least one part of the signal peptide of said GP1 subunit, and (ii), optionally, at least one tag and/or or at least one spacer.

Deleting the mucin domain advantageously allows improving the immune response by increasing the activity of the polyclonal antibodies.

A variant of a GP1 subunit may thus comprise or consist of:
- a first fragment of said GP1 subunit, wherein said fragment consists of a sequence at least 80% identical, preferably at least 85% identical, more preferably at least 90% identical, still more preferably at least 95%, 96%, 97%, 98% or 99% identical to a sequence consisting of amino acids 33 to 311 of sequence SEQ ID NO: 1,
- a second fragment of said GP1 subunit, wherein said fragment consists of a sequence at least 80% identical, preferably at least 85% identical, more preferably at least 90% identical, still more preferably at least 95%, 96%, 97%, 98% or 99% identical to a sequence consisting of amino acids 463 to 500 of sequence SEQ ID NO: 1, and
- optionally, at least one tag and/or or at least one spacer.

In one preferred embodiment, a variant of a GP1 subunit comprises or consists of (i)two fragments of said GP1 subunit, wherein the first fragment consists of amino acids 33 to 311 of sequence SEQ ID NO: 1 and wherein the second fragment consists of amino acids 463 to 500 of sequence SEQ ID NO: 1, and (ii), optionally, at least one tag and/or or at least one spacer.

The fragment of a GP1 subunit may consist of at least 50 amino acids, at least 100 amino acids, at least 150 amino acids, at least 200 amino acids, at least 250 amino acids, at least 300 amino acids, at least 350 amino acids, at least 400 amino acids or at least 450 amino acids and/or may consist of at most 100 amino acids, at most 150 amino acids, at most 200 amino acids, at most 250 amino acids, at most 300 amino acids, at most 350 amino acids, at most 400 amino acids, at most 450 amino acids or at most 490 amino acids.

A preferred variant of a GP2 subunit comprises or consists of:
- a fragment of said GP2 subunit and
- optionally, at least one tag and/or or at least one spacer,
wherein said fragment preferably lacks at least one part of the transmembrane domain of said GP2 subunit.

Deleting the transmembrane domain allows facilitating the purification of GP2 subunit variant.

A variant of a GP2 subunit may thus comprise or consist of (i) a fragment of said GP2 subunit and (ii) optionally, at least one tag and/or or at least one spacer, wherein said fragment consists of a sequence at least 80% identical, preferably at least 85% identical, more preferably at least 90% identical, still more preferably at least 95%, 96%, 97%, 98% or 99% identical to a sequence consisting of amino acids 501 to 632 of sequence SEQ ID NO: 1. In one preferred embodiment, a variant of a GP2 subunit comprises or consists of (i) a fragment of said GP2 subunit, wherein said fragment consists of amino acids 501 to 632 of sequence SEQ ID NO: 1, and (ii) optionally at least one tag and/or or at least one spacer. The fragment of a GP2 subunit may consist of at least 50 amino acids, at least 75 amino acids, at least 100 amino acids, at least 125 amino acids, or at least 150 amino acids and/or may consist of at most 100 amino acids, at most 125 amino acids, at most 150 amino acids or at most 170 amino acids.

By "fragment of a given protein", it is herein meant a sequence of consecutive amino acids of said protein.

A preferred variant of an Ebola virus GP glycoprotein comprises or consists of:
- a truncated form of said Ebola virus GP glycoprotein lacking at least one part of the mucin domain of the GP1 subunit and/or at least one part of the signal peptide of the GP1 subunit and/or at least one part of the transmembrane domain of said GP2 subunit, and
- optionally, at least one tag and/or or at least one spacer.

A preferred variant of an Ebola virus GP glycoprotein may thus comprise or consist of:
- a first fragment of the GP1 subunit, said fragment consisting of a sequence at least 80% identical, preferably at least 85% identical, more preferably at least 90% identical, still more preferably at least 95%, 96%, 97%, 98% or 99% identical to a sequence consisting of amino acids 33 to 311 of sequence SEQ ID NO: 1,
- a second fragment of the GP1 subunit, said fragment consisting of a sequence at least 80% identical, preferably at least 85% identical, more preferably at least 90% identical, still more preferably at least 95%, 96%, 97%, 98% or 99% identical to a sequence consisting of amino acids 463 to 500 of sequence SEQ ID NO: 1,
- a fragment of the GP2 subunit, said fragment consisting of a sequence at least 80% identical, preferably at least 85% identical, more preferably at least 90% identical, still more preferably at least 95%, 96%, 97%, 98% or 99% identical to a sequence consisting of amino acids 501 to 632 of sequence SEQ ID NO: 1, and

- optionally, at least one tag and/or or at least one spacer.

The size of the fragments is for example as defined above.

For example, the variant of an Ebola virus GP glycoprotein may comprise or consist of (i) a first fragment of the GP1 subunit, wherein said fragment consists of amino acids 33 to 311 of sequence SEQ ID NO: 1, (ii) a second fragment of the GP1 subunit, wherein said fragment consists of amino acids 463 to 500 of sequence SEQ ID NO: 1, (iii) a fragment of the GP2 subunit, wherein said fragment consists of amino acids 501 to 632 of sequence SEQ ID NO: 1 and (iv), optionally, at least one tag and/or or at least one spacer. Such variant may comprise or consist of sequence SEQ ID NO: 3, SEQ ID NO: 5, SEQ ID NO: 7 and/or SEQ ID NO: 11.

A preferred Ebola virus antigen according to the invention thus comprises or consists of a sequence at least 80% identical, preferably at least 85% identical, more preferably at least 90% identical, still more preferably at least 95%, 96%, 97%, 98% or 99% identical to sequence SEQ ID NO: 1, SEQ ID NO: 3, SEQ ID NO: 5, SEQ ID NO: 7, SEQ ID NO: 8, SEQ ID NO: 9 and/or SEQ ID NO: 11.

Starting from the consensus sequences SEQ ID NO: 1 or from one of its derived sequences SEQ ID NO: 3, 5, 7, 8, 9 or 11 advantageously allows obtaining polyclonal antibodies with a broader spectrum of activity.

As defined herein, an amino acid sequence "at least 80%, 85%, 90%, 95%, 96%, 97%, 98% or 99% identical" to a reference sequence may comprise mutations, such as deletions, insertions and/or substitutions compared to the reference sequence.

In case of substitutions, the substitution preferably corresponds to a conservative substitution as indicated in the Table 1 below. In a preferred embodiment, the sequence at least 80%, 85%, 90%, 95%, 96%, 97%, 98% or 99% identical to a reference sequence only differs from the reference sequence by conservative substitutions.

**Table 1**

| **Conservative substitutions** | **Type of Amino Acid** |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

In another preferred embodiment, the amino acid sequence at least 80%, 85%, 90%, 95%, 96%, 97%, 98% or 99% identical to a reference sequence corresponds to a homologous sequence derived from another Ebola virus of the same species as the reference sequence or of a different species than the reference sequence.

In a preferred embodiment, the amino acid sequence at least 80%, 85%, 90%, 95%, 96%, 97%, 98% or 99% identical to a reference sequence differs from the reference sequence by conservative substitutions and/or corresponds to a homologous sequence derived from another Ebola virus of the same species as the reference sequence or of a different species than the reference sequence.

By "a sequence at least x% identical to a reference sequence", it is intended that the sequence is identical to the reference sequence or differs from the reference sequence by up to 100-x amino acid alterations per each 100 amino acids of the reference sequence.

The alignment and the determination of the percentage of identity may be carried out manually or automatically using for instance the Needle program which is based on the Needleman and Wunsch algorithm, described in Needleman and Wunsch (1970) J. Mol Biol. 48:443-453, with for example the following parameters for polypeptide sequence comparison: comparison matrix: EBLOSUM62, gap open: 10, gap extend: 0.5, end gap penalty: false, end gap open penalty = 10, end gap extension penalty = 0.5; and the following parameters for polynucleotide sequence comparison: comparison matrix: EDNAFULL; gap open = 10, gap extend = 0.5, end gap penalty: false, end gap open penalty = 10, end gap extension penalty = 0.5.

The polyclonal antibodies according to the invention preferably bind to an Ebola virus antigen as defined above.

The polyclonal antibodies may be obtained by administering to an animal, preferably a non-human animal, at least one Ebola virus antigen as defined above and/or at least one nucleic acid comprising or consisting of a sequence encoding an Ebola virus antigen as defined above and/or at least one vector comprising said nucleic acid.

The Ebola virus antigen may be produced by a process as defined below.

### Nucleic acid encoding an Ebola virus antigen

The present invention also relates to a nucleic acid comprising or consisting of a sequence encoding an Ebola virus antigen.

The "Ebola virus antigen" is as defined above in the section of the same name.

The nucleic acid may comprise or consist of a sequence encoding an Ebola virus antigen linked to a spacer, for example a cleavage site spacer, and/or a tag.

The nucleic acid for example comprises or consists of a sequence encoding an Ebola virus GP glycoprotein, an Ebola virus GP1 subunit, an Ebola virus GP2 subunit and/or a variant thereof, said variant being preferably a soluble form of said protein(s).

The sequence encoding the Ebola virus GP glycoprotein may be at least 80% identical, preferably at least 85% identical, more preferably at least 90% identical to, still more preferably at least 95%, 96%, 97%, 98% or 99% identical to sequence SEQ ID NO: 2, SEQ ID NO: 4 or SEQ ID NO: 6. In one embodiment, the sequence encoding the Ebola virus GP glycoprotein is identical to sequence SEQ ID NO: 2, SEQ ID NO: 4 or SEQ ID NO: 6.

The nucleic acid may thus comprise or consist of a sequence at least 80% identical, preferably at least 85% identical, more preferably at least 90% identical to, still more preferably at least 95%, 96%, 97%, 98% or 99% identical to sequence SEQ ID NO: 2, SEQ ID NO: 4 or SEQ ID NO: 6. In one embodiment, the nucleic acid comprises or consists of sequence SEQ ID NO: 2, SEQ ID NO: 4 or SEQ ID NO: 6.

A nucleic sequence "at least 80%, 85%, 90%, 95%, 96%, 97%, 98% or 99% identical" to a reference sequence may comprise mutations, such as deletions, insertions and/or substitutions compared to the reference sequence.

In case of substitutions, the substitution preferably corresponds to a silent substitution or a substitution leading to a conservative substitution in the translated amino acid sequence by comparison to the reference sequence, for example as indicated in the Table 1 above.

In a preferred embodiment, the nucleic sequence at least 80%, 85%, 90%, 95%, 96%, 97%, 98% or 99% identical to a reference sequence only differs from the reference sequence by silent substitution(s) and/or substitution(s) leading to a conservative aminoacid substitution(s).

The nucleic acid encoding the Ebola virus antigen is preferably cloned into a vector.

The present invention thus also relates to a vector comprising at least one nucleic acid comprising or consisting of a sequence encoding an Ebola virus antigen.

The vector may comprise one and only one nucleic acid encoding an Ebola virus antigen.

Alternatively, the vector may comprise at least two nucleic acids (for example two, three or four nucleic acids) encoding the same Ebola virus antigen or at least two different Ebola virus antigens.

In a preferred embodiment, the vector comprises at least one nucleic acid encoding an Ebola virus antigen, wherein said Ebola virus antigen is an Ebola virus GP glyprotein, an Ebola virus GP1 subunit, an Ebola virus GP2 subunit or a variant thereof.

A preferred vector comprises a nucleic acid comprising or consisting of a sequence at least 80% identical, preferably at least 85% identical, more preferably at least 90% identical to, still more preferably at least 95%, 96%, 97%, 98% or 99% identical to sequence SEQ ID NO: 2, SEQ ID NO: 4 or SEQ ID NO: 6. In one embodiment, the vector comprises a nucleic acid comprising or consisting of sequence SEQ ID NO: 2, SEQ ID NO: 4 or SEQ ID NO: 6.

The "vector comprising at least one nucleic acid encoding an Ebola virus antigen" is thereafter also referred to as "vector".

The nucleic acid is preferably an isolated nucleic acid.

The vector is preferably an isolated vector.

An "Isolated nucleic acids" or "isolated vectors" include nucleic acid molecules purified by standard purification methods.

The term "isolated" in reference to a biological component (such as a nucleic acid, a vector or a protein) refers to a biological component that has been substantially separated or purified away from other biological components in the cell of the organism, or the organism itself, in which the component naturally occurs, such as other chromosomal and extra-chromosomal DNA and RNA, proteins, cells, and organelles. "Isolated nucleic acids" or "isolated vectors" include nucleic acid molecules purified by standard purification methods. These terms also encompass nucleic acids and vectors prepared by amplification and/or cloning, as well as chemically synthesized nucleic acids and vectors.

The vector preferably comprises at least one Ebola virus antigen expression cassette, i.e. a sequence encoding the Ebola virus antigen placed under the control of at least one expression signal allowing its expression.

The expression signal is particularly selected among a promoter, a terminator, an enhancer and their combinations.

The vector is preferably appropriate for an expression system distant from the human expression system.

In a preferred embodiment, the vector is appropriate for insect expression, more preferably for Drosophilia expression, or for mammalian expression. Suitable promoters, terminators and enhancers are well-known by the skilled person.

For example, the promoter is the Drosophila metallothionein gene promoter or a mammalian promoter.

For example, the terminator is SV40 PA terminator.

The vector may be a non-viral vector or a viral vector.

A non-viral vector may be selected in the group consisting of a plasmid, a liposomal nucleic acid complex and a carrier-associated nucleic acid.

By "plasmid", it is herein meant a double-stranded circular DNA. The plasmid may include a marker gene enabling to select the cells comprising said plasmid, an origin of replication to allow the cell to replicate the plasmid and/or a multiple cloning site allowing the insertion of a DNA fragment, in particular the sequence encoding the Ebola virus antigen.

For example, the plasmid may comprise Puromycin as a marker gene.

Non-limitative examples of carrier-associated nucleic acid are polymer-carried DNA and cationic lipids.

The liposomes used in liposomal nucleic acid complexes are well-known in the art. Said liposomes may be cationic, anionic or neutral liposomes.

Cationic lipids are also known in the art and are commonly used for gene delivery. Such lipids include Lipofectin Tm also known as DOTMA (N- [I- (2, 3-dioleyloxy) propyls N, N, N-trimethylammonium chloride), DOTAP (1, 2-bis (oleyloxy)-3 (trimethylammonio) propane), DDAB (dimethyldioctadecyl- ammonium bromide), DOGS (dioctadecylamidologlycyl spermine) and cholesterol derivatives such as DC-Chol (3 beta-(N- (N', N'- dimethyl aminomethane)-carbamoyl) cholesterol). Cationic lipids for gene delivery are preferably used in association with a neutral lipid such as DOPE (dioleyl phosphatidylethanolamine).

In another embodiment, the vector comprising a nucleic acid encoding the Ebla virus antigen is a viral vector.

By "viral vector", it is herein meant a recombinant viral vector.

The viral vector is preferably selected in the group consisting of a baculovirus vector, a retrovirus vector, a lentivirus vector, an adenovirus vector and an adeno-associated virus vector.

Baculovirus vectors are particularly suitable for insect expression.

### Method for producing an Ebola virus antigen

The present invention also relates to a method for producing an Ebola virus antigen, said antigen being as defined above in the section of the same name.

Methods for producing a protein, such as an antigen, are well-known by the skilled person.

The method for producing at least one Ebola virus antigen preferably comprises: (i) providing cells comprising (a) at least one nucleic acid sequence comprising or consisting of a sequence encoding said Ebola virus antigen or (b) at least one vector comprising at least one nucleic acid comprising or consisting of a sequence encoding said Ebola virus antigen, (ii) culturing the cells, in particular in conditions allowing the expression of said Ebola virus antigen, (iii) recovering said Ebola virus antigen.

The "nucleic acid" and the "vector" are as defined above in the section "Nucleic acid encoding an Ebola virus antigen".

The cells used in step (i) are preferably eukaryotic cells, for example insect cells such as Drosophila cells, or mammalian cells, preferably distant from human cells. The cells of step (i) may be obtained by transfection of said nucleic acid or vector.

The cells comprising said nucleic acid or vector may be selected using a selectable marker, such as puromycin.

In step (ii), the cells are cultured, in particular in conditions allowing the expression of said Ebola virus antigen. The skilled person knows the conditions of culture in function of the cells used. Besides, depending on the expression system used, the expression of the antigen may be induced by adding a specific compound in the culture medium, such as for example CuSO₄.

The nucleic acid sequence comprising or consisting of a sequence encoding said Ebola virus antigen or the vector may be integrated or not in the genome of the cells.

In a preferred embodiment, the Ebola virus antigen is a soluble and secreted protein.

When the Ebola virus antigen is a soluble and secreted protein, step (iii) may for example comprise centrifuging the cell culture obtained in step (ii) and collecting the supernatant.

Step (iii) preferably comprises:
- extracting the Ebola virus antigen, in particular from the cells and/or from the cell culture, for example using sonication, at least one cycle of freezing and thawing, homogenization by high pressure, filtration, permeabilization, cell lysis, centrifugation and/or chromatography,
- optionally further purifying said Ebola virus antigen, by precipitation, differential solubilisation, centrifugation, ultracentrifugation, proteolysis and/or chromatography,
- optionally concentrating the Ebola virus antigen, for example by lyophilization or ultrafiltration.

The steps of extracting and purifying may consist in a single step.

Methods of extracting, purifying and/or concentrating a protein are well-known by the skilled person.

For example, when the recombinant antigen comprises a Strep tag or a His tag, a Strep-Tactin resin or a Ni resin, respectively, may be used for purification.

### Polyclonal antibodies

The present invention relates to polyclonal antibodies specific for the Ebola virus.

The term "antibody" herein means an immunoglobulin molecule (also called Ig) or immunologically active portion thereof that specifically binds to an antigen.

The expression "polyclonal antibodies" herein means antibodies originating from different B lymphocyte clones, which are thus capable of binding to and/or reacting with at least two different specific epitopes on the same or on different antigens.

An "epitope" (also called "determinant" or "antigenic determinant") is the portion of a molecule that is bound by an antibody.

An antibody is generally a tetramer consisting in two identical copies of both a heavy and light chains bound by disulfide and non-covalent bonds, also called herein whole IgG. The N-terminal regions of the light and heavy chains define a variable region responsible for antigen recognition. The C-terminal regions of the two heavy chains form the Fc domain.

The light chain comprises one variable region and one constant region.

The heavy chain comprises one variable region and at least three constant regions.

Each of the light and heavy chain variable region comprises three hypervariable regions, called complementary determining regions or CDRs, which form the epitope binding site.

An antibody may also be in the form a fragment, for example produced by degradation with various peptidases, such as Fab (monovalent for antigen binding), F(ab')2 (bivalent for antigen binding) or Fc (effector domains).

The polyclonal antibodies are preferably selected from the group consisting of whole IgG, Fab, F(ab')2 and their combinations.

Antibodies comprise different classes defined according to the amino acid sequence of the heavy chain constant region. For example, mammal classes comprise IgA, IgD, IgE, IgG and IgM; avians classes comprise IgY, IgM and IgA. Subclasses may also be defined. For example, IgG and IgA are further divided into isotypes IgG1, IgG2, IgG3, IgG4, IgA1 and IgA2 in humans.

The light chain of an antibody may also be classified as kappa or lambda, based on the amino acid sequence of the light chain constant region.

The polyclonal antibodies specific for the Ebola virus according to the invention are also referred to as *"polyclonal antibodies"* in the following.

In a preferred embodiment, the polyclonal antibodies specific for an Ebola virus specifically bind to the GP glycoprotein, the GP1 subunit and/or the GP2 subunit of said Ebola virus.

The binding of the polyclonal antibodies to the Ebola virus antigen for which they are specific preferably results in blocking, completely or at least partially, the replication of the Ebola virus and/or blocking completely or at least partially, the function of the Ebola virus and/or in activating complement and/or other effector functions, thereby allowing the elimination of the Ebola virus particles.

In a preferred embodiment, the binding of the polyclonal antibodies to the Ebola virus antigen for which they are specific results in blocking, completely or at least partially, the replication of the Ebola virus. Such polyclonal antibodies are called neutralizing antibodies.

The polyclonal antibodies are preferably isolated antibodies.

The term "isolated" is as defined above.

The polyclonal antibodies are for example isolated from the blood, egg yolk, milk or colostrum of an immunized animal.

The polyclonal antibodies are preferably non-human polyclonal antibodies.

The expression "non-human polyclonal antibodies" means that the polyclonal antibodies originate from non-human animal(s). Said non-human animal is for example rabbit, mouse, rat, guinea pig, chicken, goat, horse, cow or sheep, preferably rabbit.

The non-human polyclonal antibodies may be antibodies originating from animal(s) of the same genus and/or from animals of different genus.

The polyclonal antibodies are for example selected in the group consisting of rabbit polyclonal antibodies, murine polyclonal antibodies, rat polyclonal antibodies, guinea pig polyclonal antibodies, chicken polyclonal antibodies, goat polyclonal antibodies, horse polyclonal antibodies, cow polyclonal antibodies, sheep polyclonal antibodies and their combinations.

The polyclonal antibodies according to the invention are preferably prepared by immunization of an animal, preferably a non-human animal, with at least one Ebola virus antigen as defined above and, depending on the animal, purification of the obtained polyclonal antibodies, for example from blood, egg yolk, milk or colostrum.

In one preferred embodiment, the polyclonal antibodies are obtained, in particular purified, from at least one biological sample of an animal, preferably a non-human animal, immunized with (i) at least one antigen comprising or consisting of a sequence at least 80% identical to sequence SEQ ID NO: 1, SEQ ID NO: 3, SEQ ID NO: 5, SEQ ID NO: 7, SEQ ID NO: 8, SEQ ID NO: 9 or SEQ ID NO: 11 and/or (ii) at least one nucleic acid comprising or consisting of a sequence encoding said antigen and/or (ii) a vector comprising said nucleic acid.

The polyclonal antibodies may be obtained by the method of production as defined below in the section *"Method for producing polyclonal antibodies*".

The expression "*polyclonal antibodies specific for the Ebola virus*" herein means polyclonal antibodies that are specific of at least one antigen of said Ebola virus, i.e. that specifically binds to at least one antigen of the Ebola virus. The specific binding of polyclonal antibodies to a given antigen may be assessed by ELISA, *in vitro* neutralizing assay using a mammalian (preferably human) cell line infected by Ebola virus and/or mammalian (preferably human) cells transfected with an Ebola recombinant virus and/or *in vivo* assay using an animal model infected by Ebola virus.

The animal model is preferably a rodent or primate animal model.

The antigen used in the ELISA may be the antigen used for immunizing the animals and/or another Ebola virus GP glycoprotein, for example expressed in a mammalian (preferably human) cell line transfected with a plasmid encoding said GP glycoprotein.

In the *in vitro* neutralizing assay, the Ebola recombinant virus used for transfecting the cells is for example a rVSV *(recombinant Vesicular Stomatitis Virus)* carrying Ebola virus GP glycoprotein. The Ebola recombinant virus carrying Ebola GP glycoprotein is for example incubated with the polyclonal antibodies and then with a mammalian (preferably) human cell line. Said human cell line is for example HEK293 cells or VERO cells.

The "Ebola virus" and the "antigen of the Ebola virus" are as defined above in the sections of the same name.

In a preferred embodiment, the polyclonal antibodies specifically bind to the Ebola virus GP protein, the Ebola virus GP1 protein, the Ebola virus GP2 protein and/or a variant thereof, as defined above.

In a preferred embodiment, the polyclonal antibodies specifically bind to a truncated form of the Ebola virus GP glycoprotein of sequence SEQ ID NO: 1, for example to a sequence at least 80% identical to sequence SEQ ID NO: 3 and/or a sequence encoded by a sequence at least 80% identical to sequence SEQ ID NO: 2.

The polyclonal antibodies are preferably provided in a pharmaceutical composition, particularly as defined below.

### Method for producing polyclonal antibodies

The present invention also relates to a method, preferably an *in vitro* method, for producing polyclonal antibodies specific for the Ebola virus.

Methods for producing polyclonal antibodies specific for an antigen are well-known by the skilled person.

The present invention thus relates to a method for producing polyclonal antibodies specific for the Ebola virus, wherein said method comprises purifying the polyclonal antibodies from a biological sample of an animal immunized with (i) at least one Ebola virus antigen, (ii) at least one nucleic acid comprising or consisting of a sequence encoding said Ebola virus antigen and/or (iii) at least one vector comprising said nucleic acid.

The present invention particularly relates to a method for producing polyclonal antibodies specific for the Ebola virus, said method comprising:
- providing at least one biological sample from an animal immunized with (i) at least one Ebola virus antigen, (ii) at least one nucleic acid comprising or consisting of a sequence encoding said Ebola virus antigen and/or (iii) at least one vector comprising said nucleic acid, and
- purifying the polyclonal antibodies from said biological sample.

The immunized animal is preferably non-human.

The non-human animal may be a rabbit, mouse, rat, guinea pig, chicken, goat, horse, cow or sheep, preferably rabbit.

When at least two biological samples are provided, they are preferably mixed to form a biological sample pool.

The biological sample may be any sample from the immunized animal that comprises antibodies.

Depending on the animal, the biological sample may be blood, milk, egg yolk and/or colostrum. In a preferred embodiment, the biological sample is blood.

The pool of biological samples may comprise biological samples originating from the same animal and/or from different animals, preferably of the same genus.

The Ebola virus antigen and the nucleic acid are as defined above in the section of the same name.

In one embodiment, the animal is immunized with at least one Ebola virus antigen, for example one, two, three or at least three Ebola virus antigens. Said Ebola virus antigens may belong to the same Ebola virus species or to different Ebola virus species.

The antigen used for immunization is preferably administered with one or at least one adjuvant.

Non-limitative examples of adjuvant are Freund's complete adjuvant, Freund's incomplete adjuvant, aluminum salts (such as AI(OH)3, AlPO4), Quil A, Iscoms, Montanide, TiterMax™ or RIBI™.

The antigen may be administered to the animal by the subcutaneous, intradermal, intramuscular, intraperitoneal and/or intravenous route.

The antigen may be administered to the animal as a single injection at one site or as multiple injections of low volumes at different sites, for example at two, three or four different sites.

The antigen may be administered to the animal several times at given intervals of time, for example two or three times.

In another embodiment, the animal is immunized with at least one nucleic acid comprising or consisting of a sequence encoding said Ebola virus antigen.

The nucleic acid may be provided in the form of a vector comprising said nucleic acid, said vector being as defined above.

The nucleic acid or vector is administered to the animal, so as to be expressed by cells of the animal and, preferably, secreted in the blood.

The Ebola virus antigen, administered as a protein or expressed by the cells from the administered nucleic acid or vector, thus allows triggering an immune response.

It is advantageous to monitor the presence of an immune response before collecting biological sample(s) from the animal(s).

In a preferred embodiment, the present invention relates to a method for producing polyclonal antibodies specific for the Ebola virus, said method comprising:
- providing at least one biological sample from an animal, preferably a non-human animal, immunized with (i) at least one antigen selected in the group consisting of the Ebola virus GP glycoprotein, the Ebola virus GP1 subunit, the Ebola virus GP2 subunit and a variant thereof, and/or (ii) at least one nucleic acid comprising or consisting of a sequence encoding said antigen and/or (iii) at least one vector comprising said nucleic acid, and
- purifying the polyclonal antibodies from said biological sample.

In a more preferred embodiment, the Ebola virus antigen is a variant of the Ebola virus GP glycoprotein. For example, the Ebola virus antigen comprises or consists of sequence SEQ ID NO: 3, SEQ ID NO: 5, SEQ ID NO: 7 or SEQ ID NO: 11 and/or is encoded by sequence SEQ ID NO: 2, SEQ ID NO: 4 or SEQ ID NO: 6.

The present invention particularly relates to a method for producing polyclonal antibodies specific for the Ebola virus, said method comprising:
- providing a blood sample or a pool of blood samples from an animal, preferably a non-human animal, immunized with (i) at least one Ebola virus antigen and/or (ii) at least one nucleic acid encoding said Ebola virus antigen and/or (iii) at least one vector comprising a nucleic acid encoding said Ebola virus antigen, and
- purifying the polyclonal antibodies from said blood sample or pool of blood samples.

When the biological sample is blood, the purification step preferably comprises a) clotting the blood sample or pool of blood samples and b) collecting the liquid phase, in order to obtain serum or a pool of sera.

The polyclonal antibodies are preferably further purified from said serum or pool of sera.

The step of purifying the polyclonal antibodies that are specific for the Ebola virus antigen may comprise at least one step of precipitation.

The step of purification for example comprises or consists of at least one of the following steps, for example one, two or three of the following steps: precipitation in 50% ammonium sulphate, purification on protein A column, purification on ion exchange column and/or gel filtration.

In one embodiment, the step of purification comprises or consists of precipitation in 50% ammonium sulphate, purification on protein A-sepharose column, purification on ion exchange column and a gel filtration.

In one embodiment, the polyclonal antibodies are further purified, in order to remove antibodies that are not specific for the Ebola virus and/or to remove non-immunoglobulin factors initially present in the biological sample of the immunized animal(s).

The method for producing polyclonal antibodies specific for the Ebola virus preferably further comprises at least one step of viral inactivation and/or at least one step of sterilization.

The sterilization may be performed by microfiltration and/or pasteurization.

### Pharmaceutical composition

The polyclonal antibodies according to the invention are preferably formulated into a pharmaceutical composition.

The present invention thus also relates to a pharmaceutical composition comprising polyclonal antibodies as defined above.

The pharmaceutical composition is preferable serum-free.

As used herein, the term "serum-free" means that the pharmaceutical composition does not comprise or consist of serum, plasma and/or blood. A serum-free composition may only comprise some components of the serum, in particular polyclonal antibodies.

In other words, in a serum-free composition, the polyclonal antibodies have been purified from the serum of an immunized animal.

The pharmaceutical composition preferably comprises the polyclonal antibodies and a pharmaceutically acceptable vehicle.

The polyclonal antibodies are as defined above in the section of the same name.

The pharmaceutical composition may comprise polyclonal antibodies specific for different epitopes present on the same Ebola virus antigen and/or on different antigens.

Pharmaceutical compositions comprising the polyclonal antibodies according to the invention include all compositions, wherein said polyclonal antibodies are contained in an amount effective to achieve the intended purpose, in particular to achieve complete or at least partial blocking of the replication of the Ebola virus, i.e. complete or at least partial neutralization of the Ebola virus, and/or complete or at least partial blocking of the function of the Ebola virus, , which is capable of preventing and/or treating Ebola virus disease.

The expression "pharmaceutically acceptable" is meant to encompass any carrier, which does not interfere with the effectiveness and/or the biological activity of the polyclonal antibodies and that is preferably not toxic to the subject to who they are administered.

Pharmaceutically acceptable vehicles may be prepared by any method known by those skilled in the art.

Suitable pharmaceutically acceptable vehicles may comprise excipients and auxiliaries, which facilitate processing of the active compounds into preparations which can be used pharmaceutically. Suitable pharmaceutically acceptable vehicles are described for example in Remington's Pharmaceutical Sciences (Mack Publishing Company, Easton, USA, 1985), which is a standard reference text in this field. Pharmaceutically acceptable vehicles can be routinely selected in accordance with the mode of administration, solubility and stability of the polyclonal antibodies. For example, a pharmaceutical composition for intravenous administration may include a sterile aqueous solution, which may also comprise buffer(s), diluent(s) and other suitable additive(s). The use of biomaterials and other polymers for drug delivery, as well the different techniques and models to validate a specific mode of administration, are disclosed in literature.

The polyclonal antibodies according to the invention may be formulated in a liquid pharmaceutical composition (e.g. a solution or suspension) or in a solid pharmaceutical composition (for example a pulverulent or lyophilized pharmaceutical composition).

The amount of polyclonal antibodies to be used in a pharmaceutical composition depends, for example, on the mode of administration, the dosage regimen and the type of formulation.

For example, the pharmaceutical composition may comprise from 50 mg to 500 mg, preferably from 50 mg to 400 mg, for example from 50 mg to 200 mg or from 200 mg to 500 mg of the polyclonal antibodies according to the invention, in particular for administration to a human patient.

The pharmaceutical composition is preferably suitable for subcutaneous, intradermal, intramuscular, intraperitoneal and/or intravenous administration.

In one embodiment, the pharmaceutical composition further comprises at least another anti-Ebola virus drug and/or at least another anti-Ebola virus vaccine

In another embodiment, the pharmaceutical composition comprises the polyclonal antibodies according to the invention as the only active ingredient.

Non-limitative examples of other anti-Ebola virus drug are a humanized monoclonal antibody, a RNA polymerase blocker or inhibitor, an adenosine nucleoside analogue.

Non-limitative examples of anti-Ebola vaccine are a recombinant virus vaccine, an inactivated virus, an antigen in combination with an adjuvant.

The pharmaceutical composition may be presented in an unit dosage form, for example to facilitate accurate dosing. The term "unit dosage forms" refers to physically discrete units suitable as unitary dosages for human subjects and other animals, each unit containing a pre-determined quantity of active material calculated to produce the desired therapeutic effect, in association with a suitable pharmaceutical excipient. Typical unit dosage forms include pre-filled, pre-measured ampoules or syringes of the liquid compositions. In such compositions, the polyclonal antibodies are usually a minor component, with the remainder being various vehicles or carriers and processing aids helpful for forming the desired dosing form.

The invention further provides kits comprising a pharmaceutical composition comprising the polyclonal antibodies according to the invention and instructions regarding the mode of administration. These instructions may e.g. indicate the medical indication, the route of administration, the dosage and/or the group of subjects to be treated.

### Subject to be treated

A subject in need of a treatment for preventing and/or treating Ebola virus disease may be a mammal, for example a human being or a non-human mammal.

A human being is also referred to as an "individual" or a "patient".

Said human being may be of any age, for example an infant, child, adolescent, adult, elderly people, and of any sex.

A non-human mammal is preferably a mouse, rat, cat, dog, rabbit or primate.

The subject to be treated may suffer from Ebola virus disease or be likely to suffer from Ebola virus disease.

The subject likely to suffer from Ebola virus disease may be a subject who has been or is in contact with patient(s) suffering from Ebola virus disease, for example relatives and/or health personnel.

### Prevention and/or treatment of Ebola virus disease

The present invention thus relates to the prevention and/or treatment of the Ebola virus disease.

The "Ebola virus" is as defined as above in the section of the same name.

The Ebola virus disease is for example caused by at least one virus selected in the group consisting of *Zaire Ebolavirus*, *Bundibugyo Ebolavirus*, *Reston Ebolavirus*, *Sudan Ebolavirus* and *Taï Forest Ebolavirus,* preferably caused by *Zaire Ebolavirus.*

By the expression "treatment of Ebola virus disease", it is herein meant to eliminate at least partially the Ebola virus particles, neutralize at least partially the Ebola virus particles and/or improve the symptoms resulting from the Ebola virus disease, in particular in a subject suffering from Ebola virus disease.

Desirable effects of treatment thus include:
- stopping or reducing the multiplication of the Ebola virus particles,
- inactivating, at least partially, the Ebola virus particles, for example by preventing, at least partially, the Ebola virus particles to enter the cells of the subject,
- improving or make disappear the symptom(s) of the Ebola virus, in particular fever, fatigue, muscle pain, headache, sore throat, vomiting, diarrhea, rash, kidney function, liver function, internal bleeding and/or external bleeding, and/or
- increasing the chances of survival.

By the expression "prevention of Ebola virus disease", it is herein meant to block or limit the transmission of the Ebola virus to a subject, in particular to prevent at least partially the multiplication of the Ebola virus particles, neutralize at least partially the Ebola virus particles, eliminate at least partially the Ebola virus particles and/or prevent at least partially the symptoms resulting from the Ebola virus disease, in particular in a subject likely to suffer from Ebola virus disease.

Desirable effects of prevention thus include:
- preventing at least partially the multiplication of the Ebola virus particles,
- inactivating, at least partially, the Ebola virus particles, for example by preventing, at least partially, the Ebola virus particles to enter the cells of the subject,
- preventing at least partially the appearance of Ebola virus symptom(s), in particular fever, fatigue, muscle pain, headache, sore throat, vomiting, diarrhea, rash, kidney function, liver function, internal bleeding and/or external bleeding, and/or
- increasing the chances of survival.

### Polyclonal antibodies for use in the prevention and/or treatment of Ebola virus disease and method of prevention and/or treatment of Ebola virus disease

The present invention particularly relates to the polyclonal antibodies as defined above for use in the prevention and/or treatment of Ebola virus, in particular in a subject in need thereof.

The present invention thus relates to the use of the polyclonal antibodies as defined above for the manufacture of a medicament intended for the prevention and/or treatment of Ebola virus, in particular in a subject in need thereof.

The present invention is also directed to a method for preventing and/or treating Ebola virus disease in a subject in need thereof, said method comprising a step of administering polyclonal antibodies as defined above to said subject.

The polyclonal antibodies are specific for the Ebola virus.

The polyclonal antibodies are as defined above in the section of the same name.

The polyclonal antibodies may be obtained by the method for producing polyclonal antibodies as defined above.

The polyclonal antibodies are preferably provided in the form of a pharmaceutical composition as defined above.

The present invention thus also relates to a pharmaceutical composition comprising polyclonal antibodies as defined above for use in the prevention and/or treatment of Ebola virus disease, in particular in a subject in need thereof.

The present invention also relates to a method for preventing and/or treating Ebola virus disease in a subject in need thereof, said method comprising a step of administering a pharmaceutical composition comprising the polyclonal antibodies as defined above to said subject.

The expressions "pharmaceutical composition", "Ebola virus" and "prevention and/or treatment of Ebola virus disease" are particularly as defined above in the corresponding sections of the same name.

The "subject in need thereof" is as defined above in the section "subject to be treated".

The polyclonal antibodies are preferably used or administered in an effective amount.

The expression "effective amount", also referred to as "therapeutically effective amount" is as defined above. Such effective amounts can be routinely determined by those of skilled in the art. The amount of the polyclonal antibodies actually administered will typically be determined by a physician, in the light of the relevant circumstances, including the condition to be treated, the chosen route of administration, the actual polyclonal antibodies administered, the age, sex, weight, and response of the individual subject, the severity of the subject's symptoms, and the like. It will also be appreciated by those of skilled in the art that the dosage may be dependent on the stability of the administered polyclonal antibodies.

The effective amount may also vary according to the drug or prodrug with which the polyclonal antibodies may be co-administered.

A therapeutically effective amount encompasses an amount in which any toxic or detrimental effects of the polyclonal antibodies are outweighed by the therapeutically beneficial effects.

The polyclonal antibodies or the pharmaceutical composition may be used or administered in a single dose or in several doses. Said several doses may be used or administered at the same time, separately or sequentially.

The polyclonal antibodies or pharmaceutical composition comprising thereof are preferably used or administered by subcutaneous, intradermal, intramuscular, intraperitoneal and/or intravenous route.

A preferred route is the intravenous route, in particular in a subject infected with an Ebola virus having a high virus replication rate. The intravenous route indeed allows a quick therapeutic response.

In one embodiment of the invention, a combination of polyclonal antibodies specific for the Ebola virus GP glycoprotein, the Ebola virus GP1 subunit, the Ebola virus GP2 subunit and/or a variant thereof and of polyclonal antibodies specific for at least another Ebola virus antigen may be used. Said polyclonal antibodies specific for different Ebola virus antigens may be used or administered simultaneously, separately or sequentially, in any order.

The polyclonal antibodies may be used or administered in one dose ranging from 50 mg to 500 mg of polyclonal antibodies, preferably from 50 mg to 400 mg, for example from 50 mg to 200 mg or from 200 mg to 500 mg, in particular for intravenous administration.

The polyclonal antibodies may be used or administered in one dose or several doses, said dose being for example as defined above.

When at least two doses are used or administered, they are preferably used or administered sequentially over time, for example on dose once, twice or three times a day for one day or at least two days.

For the prevention of the Ebola virus disease, the polyclonal antibodies are advantageously used or administered in one dose, preferably as soon as possible after exposition to the Ebola virus.

The polyclonal antibodies are preferably administered at least until elimination of the viral particles and/or improvement or disappearance of the symptoms, in particular fever, fatigue, muscle pain, headache, sore throat, vomiting, diarrhea, rash, kidney function, liver function, internal bleeding and/or external bleeding.

The polyclonal antibodies may be used or administered in combination with at least another anti-Ebola virus drug and/or at least another anti-Ebola virus vaccine.

Said other anti-Ebola virus drug and said other anti-Ebola virus vaccine are particularly as defined above.

The term "comprising" as used herein encompasses the term "consisting of".

The present invention will be further illustrated in view of the following examples and figures.

### Brief description of the sequences

Sequence SEQ ID NO: 1 corresponds to a consensus amino acid sequence of Ebola virus GP glycoprotein.
Sequence SEQ ID NO: 2 corresponds to a nucleic acid sequence encoding the truncated Ebola virus GP glycoprotein of sequence SEQ ID NO: 3.
Sequence SEQ ID NO: 3 corresponds to the amino acid sequence of a truncated Ebola virus GP glycoprotein consisting of amino acids 33-311 and 463-632 of sequence SEQ ID NO: 1.
Sequence SEQ ID NO: 4 corresponds to a nucleic acid sequence encoding sequence SEQ ID NO: 5.
Sequence SEQ ID NO: 5 corresponds to the amino acid sequence of the truncated Ebola virus GP glycoprotein of sequence SEQ ID NO: 3 linked to an enterokinase cleavage site and a twin strep tag.
Sequence SEQ ID NO: 6 corresponds to a nucleic acid sequence encoding sequence SEQ ID NO: 7.
Sequence SEQ ID NO: 7 corresponds to the amino acid sequence of the truncated Ebola virus GP glycoprotein of sequence SEQ ID NO: 3 linked to an enterokinase cleavage site and a his tag.
Sequence SEQ ID NO: 8 corresponds to a consensus amino acid sequence of Ebola virus GP1 subunit.
Sequence SEQ ID NO: 9 corresponds to a consensus amino acid sequence of Ebola virus GP2 subunit.
Sequence SEQ ID NO: 10 corresponds to the amino acid sequence of an enterokinase cleavage site spacer.
Sequence SEQ ID NO: 11 corresponds to the amino acid sequence SEQ ID NO: 3 linked to the amino acid sequence SEQ ID NO: 10.

### EXAMPLE: Polyclonal antibodies for use in the prevention and/or treatment of Ebola virus disease

The recombinant antigens of sequence SEQ ID NO: 5 and SEQ ID NO: 7 are produced as follows. Two pMTpuro vectors comprising either sequence SEQ ID NO: 4 or SEQ ID NO: 6 are used for transfecting Drosophilia Schneider 2(S2) cells. A stable selection of transfected cells is then performed with 6 µg/ml puromycin. Secreted recombinant antigen expression is induced with 0,5 mM CuSO₄ for 4 days. The recombinant antigens are then purified on Strep-Tactin resin (for the recombinant antigen carrying a Strep tag) or on a Ni resin (for the recombinant antigen carrying a His tag). The recombinant antigens are further purified by Superdex 200 size-exclusion chromatography in 10 mM Tris and 150 mM NaCl (pH 7,5) (1xTBS). If needed, the tag is then removed using an enterokinase acting on the cleavage site present between the truncated GP and the tag. The purified recombinant antigens comprise sequence SEQ ID NO: 11.

Rabbits are then immunized by injecting 0,1 mg of purified recombinant antigen per injection. Four injections are performed at day 10, 38, 66 and 87. Serum is collected between the injections to monitor the immunization process. The rabbits are sacrificed on day 90 and the serum is collected.

The polyclonal antibodies are purified from the serum by precipitation in 50% ammonium sulphate followed by purification on protein A-sepharose column, purification ion exchange column, gel filtration and sterilization by microfiltration and pasteurization.

The specific binding of the polyclonal antibodies to the recombinant antigen comprising sequence SEQ ID NO: 11 is then assessed by ELISA and the neutralizing capacity of the polyclonal antibodies is assessed in infected human cells..

## Claims

1. Polyclonal antibodies for use in a method for the prevention and/or treatment of Ebola virus disease, wherein said polyclonal antibodies are specific for the Ebola virus.

2. The polyclonal antibodies for use according to claim 1, wherein said polyclonal antibodies are non-human polyclonal antibodies.

3. The polyclonal antibodies for use according to claim 1 or 2, wherein said polyclonal antibodies specifically bind to Ebola virus GP glycoprotein, Ebola virus GP1 subunit, Ebola virus GP2 subunit and/or a variant thereof.

4. The polyclonal antibodies for use according to claim 3, wherein said variant comprises or consists of a sequence at least 80% identical to sequence SEQ ID NO: 3, SEQ ID NO: 5, SEQ ID NO: 7 and/or SEQ ID NO: 11.

5. The polyclonal antibodies for use according to claim 3 or 4, wherein said variant is encoded by a nucleic acid comprising or consisting of a sequence at least 80% identical to sequence SEQ ID NO: 2, SEQ ID NO: 4 and/or SEQ ID NO: 6.

6. The polyclonal antibodies for use according to any one of claims 1 to 5, wherein said polyclonal antibodies are purified from at least one biological sample of an animal immunized with (i) at least one antigen selected in the group consisting of Ebola virus GP glycoprotein, Ebola virus GP1 subunit, Ebola virus GP2 subunit and a variant thereof, (ii) at least one nucleic acid comprising or consisting of a sequence encoding said antigen and/or (iii) at least one vector comprising at least one nucleic acid comprising or consisting of a sequence encoding said antigen.

7. The polyclonal antibodies for use according to any one of claims 1 to 6, wherein said polyclonal antibodies are provided in a pharmaceutical composition.

8. The polyclonal antibodies for use according to claim 7, wherein said pharmaceutical composition is suitable for subcutaneous, intradermal, intramuscular, intraperitoneal and/or intravenous administration.

9. The polyclonal antibodies for use according to claim 7 or 8, wherein said pharmaceutical composition comprises from 50 mg to 500 mg of said polyclonal antibodies.

10. The polyclonal antibodies for use according to any one of claims 1 to 9, wherein said polyclonal antibodies are used in combination with at least another anti-Ebola virus drug and/or at least another anti-Ebola virus vaccine.

11. Polyclonal antibodies specific for the Ebola virus obtained from at least one biological sample of an animal immunized with (i) at least one antigen comprising or consisting of a sequence at least 80% identical to sequence SEQ ID NO: 1, SEQ ID NO: 3, SEQ ID NO: 5, SEQ ID NO: 7, SEQ ID NO: 8, SEQ ID NO: 9, or SEQ ID NO: 11, (ii) at least one nucleic acid comprising or consisting of a sequence encoding said antigen and/or (iii) at least one vector comprising at least one nucleic acid comprising or consisting of a sequence encoding said antigen.

12. An antigen comprising or consisting of sequence SEQ ID NO: 1, sequence SEQ ID NO: 8, sequence SEQ ID NO: 9 or a sequence at least 80% identical to sequence SEQ ID NO: 3, SEQ ID NO: 5, SEQ ID NO: 7 or SEQ ID NO: 11.

13. A nucleic acid comprising or consisting of a sequence encoding an antigen according to claim 12.

14. A vector comprising a nucleic acid according to claim 13.

15. A method for producing polyclonal antibodies, wherein said method comprises:
- providing at least one biological sample from an animal immunized with (i) at least one antigen comprising or consisting of a sequence at least 80% identical to sequence SEQ ID NO: 1, SEQ ID NO: 3, SEQ ID NO: 5, SEQ ID NO: 7, SEQ ID NO: 8, SEQ ID NO: 9 or SEQ ID NO: 11 and/or (ii) at least one nucleic acid comprising or consisting of a sequence encoding said antigen and/or (iii) at least one vector comprising a nucleic acid comprising or consisting of a sequence encoding said antigen, and
- purifying the polyclonal antibodies from said biological sample.
